# FASCICULE DE BREVET EUROPEEN

(11) **EP 3 237 072 B1**
(45) Date de publication et mention de la délivrance du brevet: **10.07.2024**
(21) Numéro de dépôt: 15832817.9
(22) Date de dépôt: 22.12.2015
(51) Int. Cl.: A61Q 19/06, A61K 8/368, A61K 8/97, A61Q 19/08, A61K 8/9789, A61P 17/00

(54) **UTILISATION D'UN EXTRAIT DE LYTHRUM SALICARIA**
VERWENDUNG EINES EXTRAKTES VON LYTHRUM SALICARIA
USE OF AN EXTRACT OF LYTHRUM SALICARIA

(30) Priorité: 23.12.2014 FR 1463205
(43) Date de publication de la demande: 01.11.2017
(73) Titulaire: BASF Beauty Care Solutions France SAS, 69007 Lyon (FR)
(72) Inventeur: CITTADINI, Lysianne, 01700 Miribel (FR); DANOUX, Louis, 54420 Saulxures-Les-Nancy (FR); GODARD, Nathalie, 69610 Aveize (FR)
(74) Mandataire: Cabinet Beau de Loménie
(86) Numéro de dépôt international: PCT/FR2015/053700
(87) Numéro de publication internationale: WO 2016/102874

(56) Documents cités:
- KR-A- 20130 060 446
- RO-A2- 126 901
- MR. PLANTES: "Salicaire (Lythrum salicaria)", 8 April 2011 (2011-04-08), XP002743400, Retrieved from the Internet <URL:http://www.mr-plantes.com/2011/04/salicaire-lythrum-salicaria/> [retrieved on 20150806]
- TUNALIER ET AL: "Antioxidant, anti-inflammatory, anti-nociceptive activities and composition of Lythrum salicaria L. extracts", JOURNAL OF ETHNOPHARMACOLOGY, ELSEVIER IRELAND LTD, IE, vol. 110, no. 3, 13 March 2007 (2007-03-13), pages 539 - 547, XP005931840, ISSN: 0378-8741, DOI: 10.1016/J.JEP.2006.10.024
- EUNSON HWANG ET AL: "Gallic Acid Regulates Skin Photoaging in UVB-exposed Fibroblast and Hairless Mice", PHYTOTHERAPY RESEARCH., vol. 28, no. 12, 8 December 2014 (2014-12-08), GB, pages 1778 - 1788, XP055276665, ISSN: 0951-418X, DOI: 10.1002/ptr.5198

## Description

La présente invention concerne l'utilisation non thérapeutique d'un extrait de la plante *Lythrum salicaria* , pour maintenir et/ou augmenter l'expression de collagène dans la peau et/ou les muqueuses notamment pour en maintenir et/ou en augmenter la fermeté et/ou pour augmenter la lipolyse dans les adipocytes de la peau, en particulier pour induire un effet amincissant et/ou pour diminuer les aspects inesthétiques de la cellulite tel que l'aspect de peau d'orange de la peau et/ou de la culotte de cheval et/ou pour le traitement de la cellulite. La présente invention concerne également l'utilisation dermatologique d'un tel extrait ou d'un des dérivés d'acide gallique choisi dans le groupe constitué par la vescalagine, la castalagine et leur mélange pour le traitement des pathologies cutanées impliquant une diminution d'expression de collagène . Elle concerne de plus l'utilisation non thérapeutique d'un dérivé d'acide gallique choisi dans le groupe constitué par la vescalagine, la castalagine et leur mélange pour induire un effet amincissant au niveau de la peau et/ou diminuer, prévenir et/ou traiter les manifestations inesthétiques de la cellulite comme par exemple l'aspect peau d'orange de la peau ou la culotte de cheval et/ou pour le traitement de la cellulite.

Le collagène est une protéine constitutive de la matrice extra-cellulaire présente dans les tissus des vertébrés, faisant partie d'une famille protéique comprenant 29 types différents.

Parmi les différents types de collagène, on retrouve le collagène de type I dans de nombreux tissus humains tels que tendons, ligaments, cornée et peau. Le collagène de type I est le collagène majoritaire de la peau. Il fait partie des collagènes fibrillaires avec le collagène III et V.

Le collagène fibrillaire est formé à partir d'un précurseur, le procollagène, qui après une étape de maturation en tropocollagène va s'assembler spontanément en fibrilles qui ensuite vont former des fibres et enfin des faisceaux pouvant mesurer 1 à 10µM de diamètres. Ces faisceaux sont stabilisés par réticulation.

C'est le collagène qui confère aux tissus leur résistance mécanique, participant ainsi de façon prépondérante au maintien de leur fermeté.

Différentes variations physiologiques au cours de la vie telles qu'un changement de régime alimentaire ou des régimes alimentaires successifs, des changements hormonaux durant la puberté, la grossesse, la ménopause, vont entrainer des modifications des tissus cutanés, induisant une perte de fermeté et un relâchement des tissus. Cette perte de fermeté implique une diminution du taux de collagène. Plusieurs mécanismes concomitants sont impliqués dans la diminution de ce taux de collagène: une diminution de la prolifération des fibroblastes dermiques, une diminution de la synthèse de collagène et la dégradation du collagène est augmentée par les métalloprotéinases, en particulier les collagénases, enzymes responsables de la dégradation des collagènes.

Ainsi, la protéine de collagène est une cible privilégiée des recherches et innovations dans le domaine de la cosmétique et de la dermo-cosmétique mais aussi nutraceutique, et de nombreuses compositions existent sur le marché visant à raffermir les tissus cutanés et améliorer la fermeté de la peau.

Il existe donc un besoin constant d'identifier de nouveaux ingrédients actifs capables de maintenir et/ou d'augmenter la fermeté des tissus, en particulier de la peau.

En outre, un besoin spécifique existe dans le domaine cosmétique et dermatologique pour la mise au point d'ingrédients actifs amincissants et/ou agissant sur la cellulite et/ou diminuant l'effet peau d'orange de la peau, en particulier chez les femmes. En effet, les variations physiologiques non pathologiques précédentes telles qu'un changement de régime alimentaire, les variations hormonales, en particulier durant la puberté, la grossesse, la ménopause mais aussi une mauvaise alimentation, la sédentarité, sont autant de causes de prise de poids et/ou d'apparition de cellulite, aussi appelée *adiposis edematosa.* La cellulite est associée à des manifestations inesthétiques telles que l'apparition d'un effet capitonné de la peau dit « peau d'orange » localisé particulièrement au niveau des cuisses, des hanches, de la taille et des fesses chez la femme, et/ou de la culotte de cheval lorsque la cellulite est localisée en haut des cuisses, sur les fesses et les hanches, et/ou de la rétention d'eau, générant un sentiment d'inconfort esthétique.

La Matrice Extra-Cellulaire (MEC), structure de soutien des tissus et contenant du collagène, est aussi présente entre les cellules du tissu conjonctif adipeux, les adipocytes.

Par conséquent, l'identification d'ingrédients cosmétiques capables à la fois d'agir sur le maintien de la fonctionnalité de la MEC en particulier via l'expression de collagène mais également capables d'amincir et/ou de diminuer les aspects inesthétiques de la peau d'orange est d'un grand intérêt cosmétique et dermatologique. Et c'est précisément le problème auquel répond la présente invention.

De façon très surprenante, les inventeurs ont en effet découvert qu'un extrait de la plante *Lythrum salicaria,* permet de maintenir et/ou augmenter l'expression de collagène dans la peau et/ou les muqueuses et ainsi de maintenir et/ou augmenter leur fermeté. Ils ont également découvert que ledit extrait, et les dérivés d'acide gallique que sont la vescalagine et la castalagine, permettent en outre d'augmenter la lipolyse dans les adipocytes de la peau et donc qu'ils ont un effet amincissant au niveau de la peau. L'extrait de la plante *Lythrum salicaria* selon l'invention et les dérivés d'acide gallique, la vescalagine et la castalagine, par leur propriétés conviennent ainsi tout particulièrement pour diminuer, prévenir et/ou traiter les manifestations inesthétiques de la cellulite comme par exemple l'aspect peau d'orange et/ou la culotte de cheval et/ou la rétention d'eau.

L'invention présente l'avantage de fournir un nouvel ingrédient actif sur le maintien et/ou l'augmentation de l'expression de collagène permettant de maintenir et/ou d'augmenter la fermeté des tissus, notamment de la peau humaine, faisant de cet ingrédient actif un ingrédient particulièrement approprié comme ingrédient actif sur les tissus cutanés distendus, relâchés. L'extrait selon l'invention, et les dérivés d'acide gallique, la vescalagine et la castalagine, permettent par ailleurs de maintenir et/ou augmenter la lipolyse dans les adipocytes de la peau, ce qui en font un nouvel ingrédient amincissant particulièrement efficace pour diminuer, prévenir et/ou traiter les manifestations inesthétiques de la cellulite comme par exemple l'aspect peau d'orange et/ou la culotte de cheval et/ou la rétention d'eau. Ainsi, l'action de l'extrait selon l'invention est double et ainsi d'un grand intérêt cosmétique et, dermatologique. Il permet en outre de diminuer le nombre d'ingrédients dans les compositions cosmétiques et dermatologiques.

Un autre avantage de la présente invention est que l'extrait selon l'invention est un extrait de plante naturel non toxique, topiquement acceptable, facilement formulable, et qui peut être produit à l'échelle industrielle dans des conditions pleinement satisfaisantes de développement durable. En outre les dérivés d'acide gallique, la vescalagine et la castalagine, sont susceptibles d'être obtenus (avantageusement sont obtenus) à partir d'un tel extrait (par exemple par purification).

L'extrait selon l'invention est un extrait de la plante *Lythrum salicaria,* communément appelée salicaire commune ou herbe aux coliques. Il s'agit d'une plante de la famille des Lythraceae très répandue en Europe, y compris en France, facilement reconnaissable à ses fleurs roses à violettes en forme d'épi. C'est une plante connue pour ses propriétés antidiarréhiques, toniques et hémostatiques. Elle possède par ailleurs des propriétés adoucissantes et apaisantes et est active sur certaines affections de la peau telles qu'eczéma et intertrigo.

La demande de brevet KR100852737 décrit un effet antioxydant ainsi qu'un effet inhibiteur sur le développement de la fibrose hépatique d'un extrait de racine de *Lythrum salicaria,* tandis que la demande CN103861030 décrit une combinaison de plusieurs plantes, dont *Lythrum salicaria,* pour son effet dans le traitement du psoriasis.

La plante *Lythrum salicaria* est aussi connue en cosmétique en tant qu'agent colorant la peau dans la demande FR2883749 déposée par la demanderesse et dans la demande FR2835183.

Par ailleurs, la demande WO2015140470 décrit l'utilisation d'acide gallique et de dérivés d'acide gallique pouvant provenir de la plante *Lythrum salicaria,* comme ingrédient actif dans une composition cosmétique, pour stimuler ou réparer la fonction barrière de l'épiderme, pour lutter contre le vieillissement de la peau, pour l'hydrater et lutter contre les agressions externes liées à la pollution et au stress chez les peaux sèches à tendance atopique.

En outre, la demande de brevet JP2003002820 décrit l'utilisation cosmétique d'un extrait de *Lythrum salicaria* pour son activité anti-élastase au niveau de la peau.

Toutefois, ni cette demande ni aucun art antérieur ne décrit les propriétés de maintien et/ou d'augmentation de l'expression protéique de collagène, ni de maintien et/ou d'augmentation de la fermeté de la peau et/ou des muqueuses.

En outre, la demande KR20130060446 décrit une composition thérapeutique comprenant une combinaison d'un extrait de *Lythrum salicaria* et d'un extrait de la plante *Aceriphyllum rossii* pour lutter contre l'obésité.

D'autre part, il est connu que *Lythrum salicaria* est un agent tonique. Toutefois, on distingue au sens de la présente invention un agent dit tonique qui permet de resserrer les pores de la peau, donc de tonifier les tissus en apportant un effet immédiat non durable de fermeté, de l'extrait de Lythrum salicaria selon la présente invention qui permet d'augmenter la fermeté de la peau par augmentation de l'expression de collagène. Enfin, la demande de brevet RO126901 divulgue l'utilisation d'un extrait de Lythrum salicaria pour le traitement de la couperose. De même, Tunalier et al. (2007) divulgue l'utilisation de l'extrait de Lythrum salicaria dans le traitement des varices.

L'article d'Eunson Hwang et al (Phytotherapy research, 28: 1778-1788 (2014)) décrit l'utilisation de l'acide gallique sur les rides, en tant qu'antioxidant et sur le photovieillissement de la peau.

Aucun art antérieur ne décrit toutefois l'utilisation d'un extrait de *Lythrum salicaria* et/ou des dérivés d'acide gallique, vescalagine et/ou castalagine, pour augmenter la lipolyse dans les adipocytes de la peau pour induire un effet amincissant au niveau de la peau et/ou diminuer les manifestations inesthétiques de la cellulite tel que l'aspect peau d'orange et/ou la culotte de cheval. Aucun art antérieur ne décrit par ailleurs l'extrait selon l'invention pour son utilisation dermatologique pour le traitement de la cellulite.

Ainsi, la présente invention a pour premier objet l'utilisation non thérapeutique d'un extrait de la plante *Lythrum salicaria* pour maintenir et/ou augmenter l'expression de collagène dans la peau et/ou les muqueuses, l'extrait de *Lythrum salicaria* étant appliqué par voie topique sur une zone de peau et/ou de muqueuse saine. La présente invention a ainsi également pour objet l'utilisation non thérapeutique d'un extrait de la plante *Lythrum salicaria* pour maintenir et/ou augmenter la fermeté de la peau et/ou des muqueuses, en particulier chez l'être humain, l'extrait de *Lythrum salicaria* étant appliqué par voie topique sur une zone de peau et/ou de muqueuse saine.

La présente invention a également pour objet l'utilisation non thérapeutique d'un extrait de la plante *Lythrum salicaria* pour maintenir et/ou augmenter la lipolyse dans les adipocytes de la peau et/ou pour induire un effet amincissant au niveau de la peau et/ou pour diminuer, prévenir et /ou traiter les manifestations inesthétiques de la cellulite comme par exemple l'aspect peau d'orange de la peau, et/ou pour la prévention et/ou le traitement de la cellulite, l'extrait de *Lythrum salicaria* étant appliqué par voie topique sur une zone de peau et/ou de muqueuse saine.

L'extrait de *Lythrum salicaria* selon l'invention contient un des dérivés d'acide gallique, en particulier de la vescalagine et/ou de la castalagine. Un objet de l'invention concerne donc l'utilisation non thérapeutique d'un des dérivés d'acide gallique choisi dans le groupe constitué par la vescalagine, la castalagine et leur mélange, préférentiellement la castalagine, avantageusement susceptible d'être obtenu, préférentiellement obtenus, à partir de la plante *Lythrum salicaria,* en particulier par extraction de cette plante, pour maintenir et/ou augmenter la lipolyse dans les adipocytes de la peau et/ou pour induire un effet amincissant au niveau de la peau et/ou pour diminuer, prévenir et /ou traiter les manifestations inesthétiques de la cellulite comme par exemple l'aspect peau d'orange de la peau et/ou la culotte de cheval, et/ou pour la prévention et/ou le traitement de la cellulite.

Au sens de la présente invention, on entend par « utilisation cosmétique » une utilisation non thérapeutique, c'est-à-dire une utilisation telle que l'extrait de *Lythrum salicaria* et/ou un des dérivés d'acide gallique choisi dans le groupe constitué par la vescalagine, la castalagine et leur mélange, plus préférentiellement la castalagine, est appliqué sur une zone de peau et/ou de muqueuse saine.

Au sens de la présente invention, on entend par « zone de peau et/ou de muqueuse saine » une zone de peau et/ou de muqueuse qualifiée de non pathologique par un dermatologue, c'est-à-dire qui ne présente pas d'infection, de maladie ou d'affection cutanée telle que candidose, impétigo, psoriasis, eczéma, acné ou dermatite, de plaies ou de blessures ni de maladie liée à un dysfonctionnement de la lipolyse dans les tissus adipeux, particulièrement dans les adipocytes, telle que l'obésité, ni de varice, de couperose ou de télangiectasie.

De façon particulière, on entend par une « zone de peau et/ou de muqueuse saine » une zone de peau et/ou de muqueuse constituée de cellules qualifiées de « normales » par un médecin, c'est-à-dire de cellules non cancéreuses.

Au sens de la présente invention, on entend par « muqueuse » la muqueuse oculaire, la muqueuse vaginale, la muqueuse uro-génitale et/ou la muqueuse buccale, notamment buccale labiale et/ou la muqueuse gingivale, préférentiellement, les muqueuses oculaires et/ou buccales, et encore préférentiellement, la muqueuse labiale et/ou oculaire.

Au sens de la présente invention, on entend par « peau », la peau du visage et du corps incluant le cuir chevelu et par « cuir chevelu » les zones temporales du cuir chevelu, la zone frontale, la zone pariétale et le vertex ou sommet du crâne. Avantageusement il s'agit de la peau du corps.

Ainsi, avantageusement l'extrait de *Lythrum salicaria* et/ou un des dérivés d'acide gallique choisi dans le groupe constitué par la vescalagine, la castalagine et leur mélange, plus préférentiellement la castalagine, est appliqué, en particulier par massage avec des mouvements circulaires, sur au moins une zone de peau et/ou de muqueuse choisie parmi le ventre, les bras, les avant-bras, les genoux, le buste, les cuisses, les hanches, les fesses, le cou, la taille et/ou le visage, et préférentiellement le corps, en particulier, sur une zone de peau comprenant de la cellulite et/ou ses manifestations inesthétiques telles que la peau d'orange ou la culotte de cheval et/ou sur une zone de peau manquant de fermeté en raison de variations physiologiques non pathologiques telles qu'un changement de régime alimentaire, les variations hormonales, en particulier durant la puberté, la grossesse, la ménopause mais aussi une mauvaise alimentation, la sédentarité et plus particulièrement chez la femme.

Plus avantageusement, l'extrait de *Lythrum salicaria* et/ou un des dérivés d'acide gallique choisi dans le groupe constitué par la vescalagine, la castalagine et leur mélange, plus préférentiellement la castalagine, est appliqué sur une zone de peau qui n'est pas sèche et/ou à tendance atopique et/ou dont la fonction barrière de l'épiderme n'est pas altérée et/ou dont le relâchement cutané n'est pas dû à un effet du vieillissement extrinsèque ou intrinsèque (chronobiologique ou photo-induit).

L'extrait selon l'invention et/ou un des dérivés d'acide gallique choisi dans le groupe constitué par la vescalagine, la castalagine et leur mélange, est administré par voie topique. Ainsi, l'extrait selon l'invention et/ou un des dérivés d'acide gallique choisi dans le groupe constitué par la vescalagine, la castalagine et leur mélange, plus préférentiellement la castalagine, est topiquement acceptable. Au sens de la présente invention, on entend par «topiquement acceptable», un extrait et/ou un des dérivés d'acide gallique choisi dans le groupe constitué par la vescalagine, la castalagine et leur mélange, plus préférentiellement la castalagine, non toxique, non irritant pour la peau et/ou les muqueuses, n'induisant pas de réponse inflammatoire, de façon particulière pas de réponse allergique, et stable chimiquement.

Au sens de la présente invention, on entend par « utilisation topique » ou « voie topique », l'utilisation de l'extrait selon l'invention et/ou d'un des dérivés d'acide gallique choisi dans le groupe constitué par la vescalagine, la castalagine et leur mélange, plus préférentiellement la castalagine, par application locale directe, tel que par massage avec des mouvements circulaires, et/ou vaporisation de l'ingrédient sur la surface de la zone concernée de la peau et/ou des muqueuses.

Au sens de la présente invention, on entend par « collagène » le collagène de type I et/ou III et/ou IV et/ou V et/ou VI et/ou VII et/ou XII et/ou XIII et/ou XIV et/ou XVI et/ou XVII et/ou XXIV et/ou XXIX, en particulier présent dans la peau et/ou les muqueuses. Préférentiellement, il s'agit du collagène de type I et/ou III et/ou V, en particulier présent dans la peau et/ou les muqueuses, et plus préférentiellement du collagène de type I, en particulier présent dans la peau et/ou les muqueuses, en particulier humain.

L'acide gallique a la formule suivante (Masse moléculaire = 170,12 g/mol ; numéro CAS 149-91-7):

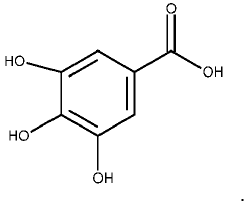

Au sens de la présente invention, on entend par « dérivés de l'acide gallique » les ellagitanins choisi dans le groupe constitué par la vescalagine, la castalagine, et leur mélange.

La vescalagine a la formule suivante (masse moléculaire = 934,07 g/mol ; numéro CAS 36001-47-5):

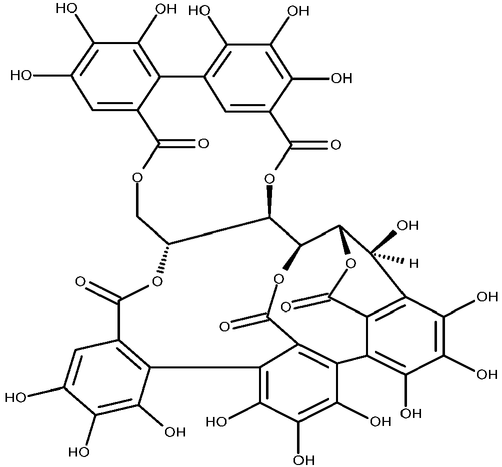

La castalagine a la formule suivante (masse moléculaire = 934,63 g/mol ; numéro CAS 24312-00-3):

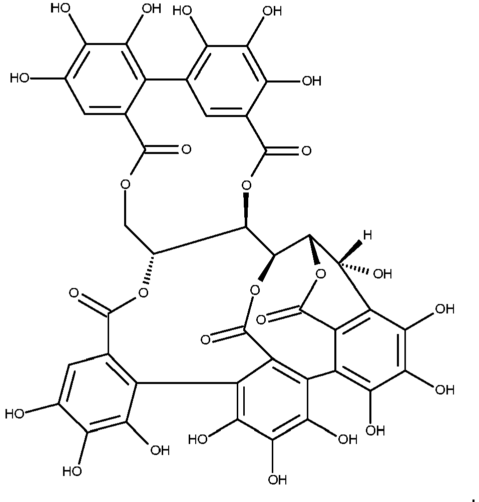

Ainsi, un objet de l'invention concerne l'utilisation non thérapeutique d'un des dérivés d'acide gallique choisi dans le groupe constitué par la vescalagine, la castalagine et leur mélange, plus préférentiellement la castalagine, plus particulièrement susceptible d'être obtenu, préférentiellement obtenu, à partir de l'extrait de Lythrum salicaria selon l'invention pour maintenir et/ou augmenter la lipolyse dans les adipocytes de la peau et/ou pour induire un effet amincissant au niveau de la peau et/ou pour diminuer, prévenir et/ou traiter les manifestations inesthétiques de la cellulite comme par exemple l'aspect peau d'orange de la peau et/ou la culotte de cheval.

Dans un mode préférentiel de réalisation de l'invention, l'utilisation non thérapeutique est l'utilisation d'un extrait de *Lythrum salicaria* comprenant un des dérivés d'acide gallique choisi dans le groupe constitué par la vescalagine la castalagine et leur mélange, plus préférentiellement la castalagine, pour maintenir et/ou augmenter l'expression de collagène dans la peau et/ou les muqueuses, pour maintenir et/ou augmenter la fermeté de la peau et/ou des muqueuses ainsi que pour maintenir et/ou augmenter la lipolyse dans les adipocytes de la peau et/ou pour induire un effet amincissant au niveau de la peau et/ou pour diminuer, prévenir et /ou traiter les manifestations inesthétiques de la cellulite comme par exemple l'aspect peau d'orange de la peau et/ou la culotte de cheval.

Au sens de la présente invention, on entend par « augmenter l'expression de collagène » l'augmentation de l'expression génique, c'est-à-dire des ANRm, et/ou l'expression protéique de collagène, préférentiellement l'augmentation de l'expression protéique de collagène, et plus préférentiellement de collagène de type I, en particulier au niveau de la peau et/ou des muqueuses, avantageusement de collagène de type I humain.

Au sens de la présente invention, on entend par « maintenir l'expression de collagène » empêcher l'expression de collagène de diminuer. Avantageusement toutefois, au sens de la présente invention, maintenir l'expression de collagène n'est pas empêcher la dégradation de collagène, notamment par celle induite par l'action des métalloprotéinases, en particulier des métalloprotéinases 1 et 3 (MMP1 et MMP3).

Dans un mode de réalisation de l'invention, l'extrait et/ou un des dérivés d'acide gallique choisi dans le groupe constitué par la vescalagine, la castalagine et leur mélange, plus préférentiellement la castalagine, est considéré comme en quantité efficace pour augmenter l'expression génique et/ou protéique de collagène dans des fibroblastes cultivés *in vitro,* lorsque l'augmentation génique et/ou protéique est d'au moins 20%, préférentiellement d'au moins 60%, plus préférentiellement d'au moins 80%, encore plus préférentiellement d'au moins 90% par rapport au niveau d'expression génique et/ou protéique de collagène mesurée dans les fibroblastes cultivés en l'absence de l'extrait, et des dérivés d'acide gallique, la vescalagine et la castalagine, plus préférentiellement la castalagine. Dans un mode préférentiel de réalisation de l'invention, il s'agit de l'augmentation de l'expression protéique de collagène de type I mesurée par technique immunochimique dans des fibroblastes humains cultivés en présence de l'extrait préparé selon l'exemple 1a), selon le protocole décrit dans l'exemple 2.

Dans un mode alternatif de réalisation de l'invention, il s'agit de l'augmentation de l'expression protéique de collagène de type I mesurée par technique immunochimique dans des fibroblastes humains cultivés en présence de l'extrait préparé selon l'exemple 1g).

Avantageusement, la mesure par technique immunochimique s'effectue à l'aide d'un anticorps anti-collagène I, selon le protocole décrit dans l'exemple 2.

Avantageusement au sens de la présente invention, les fibroblastes humains mis en culture sont normaux, c'est-à-dire non pathologiques, non cancéreux et ne nécessitent par conséquent aucun traitement thérapeutique.

Au sens de la présente invention, on entend par « augmenter la fermeté » une augmentation à des fins esthétiques de la fermeté de la peau et/ou des muqueuses qui ont perdu de la fermeté notamment sous l'effet de variations physiologiques non pathologiques telles qu'un changement de régime alimentaire, les variations hormonales, en particulier durant la puberté, la grossesse, la ménopause, l'andropause, une mauvaise alimentation ou la sédentarité, plus avantageusement qui ont perdu de la fermeté sous l'effet des variations physiologiques non pathologiques telles qu'un changement de régime alimentaire, les variations hormonales, en particulier durant la puberté, la grossesse, la ménopause, l'andropause, une mauvaise alimentation ou la sédentarité. Cette perte de fermeté peut également apparaitre sous l'effet de facteurs extrinsèques tels que les agents agressifs de l'environnement comme par exemple les radiations UV, la pollution, les fumées, le tabac, les toxines, les agressions climatiques et/ou mécaniques. Au sens de la présente invention, on entend par « maintenir la fermeté » empêcher le relâchement des tissus cutanés, de la peau et/ou des muqueuses.

Avantageusement selon l'invention, l'augmentation de la fermeté de la peau et/ou des muqueuses n'est pas concomitante à une augmentation de l'élasticité de la peau et/ou des muqueuses. On distingue en effet au sens de la présente invention, la fermeté de la peau, en particulier étroitement liée à la synthèse de collagène, plus particulièrement du collagène de type I, protéine conférant sa résistance mécanique à la matrice extra-cellulaire, et l'élasticité de la peau, en particulier dépendante de la quantité de fibres élastiques fonctionnelles.

La présente invention exclut donc l'utilisation de l'extrait de *Lythrum salicaria* et/ou d'un des dérivés d'acide gallique choisi dans le groupe constitué par la vescalagine, la castalagine et leur mélange, pour maintenir et/ou augmenter l'élasticité de la peau et/ou des muqueuses, en particulier pour inhiber l'activité des élastases présentes au niveau de la peau et/ou des muqueuses.

Par ailleurs, les méthodes de mesure classiques de la fermeté de la peau consistent à mesurer la profondeur de ses déformations possibles, tandis que celles visant à mesurer l'élasticité de la peau consistent à mesurer sa capacité à revenir à son état initial après torsion et/ou déformation. L'augmentation de la fermeté peut être mesurée selon les méthodes classiques notamment par mesure *in vivo* à l'aide d'un cutomètre, d'un densiscore, d'un torquemètre ou encore d'un dynaskin associé à un dermatop. L'augmentation de l'élasticité peut être mesurée selon les méthodes classiques notamment par mesure *in vivo*, à l'aide d'un ballistomètre ou d'un cornéovacuomètre.

L'extrait selon l'invention est particulièrement intéressant pour raffermir le corps.

Au sens de la présente invention, on entend par « augmenter la lipolyse dans les adipocytes de la peau », une augmentation de l'activité lipolytique mesurée dans des adipocytes cultivés *in vitro* en présence de l'extrait de Lythrum salicaria selon l'invention et/ou d'un des dérivés d'acide gallique choisi dans le groupe constitué par la vescalagine, la castalagine et leur mélange, plus préférentiellement de la castalagine, d'au moins 30%, préférentiellement d'au moins 35%, en particulier d'au moins 40%, encore plus préférentiellement d'au moins 70%, par rapport à l'activité lipolytique mesurée dans les adipocytes cultivés *in vitro* en l'absence de l'extrait selon l'invention et d'un des dérivés d'acide gallique choisi dans le groupe constitué par la vescalagine, la castalagine et leur mélange, plus préférentiellement de la castalagine.

Dans un mode de réalisation préféré, l'augmentation de l'activité lipolytique est mesurée au niveau d'adipocytes obtenus à partir d'une différentiation de préadipocytes normaux, c'est-à-dire non cancéreux, d'un humain sain, c'est-à-dire ne présentant aucun signe d'obésité tel que défini par un médecin, cultivés *in vitro* dans les conditions décrites dans l'exemple 3a). Avantageusement, l'activité lipolytique est mesurée par quantification du glycérol par mesure de la densité optique à 340 nm selon l'exemple 3a).

Dans un mode alternatif de réalisation, l'augmentation de l'activité lipolytique est mesurée au niveau d'adipocytes obtenus à partir d'une différentiation de préadipocytes murins normaux, c'est-à-dire non cancéreux, cultivés *in vitro* dans les conditions décrites dans l'exemple 3b) en présence de l'extrait de *Lythrum salicaria* comprenant de la vescalagine et de la castalagine préparé selon l'exemple 1g).

Au sens de la présente invention, on entend par « maintenir la lipolyse dans les adipocytes de la peau », empêcher la diminution de la lipolyse dans les adipocytes de la peau

Au sens de la présente invention, on entend par « induire un effet amincissant au niveau de la peau », une diminution visuellement observable du volume de la silhouette, préférentiellement du tour de taille et/ou du tour de hanche et/ou du tour des cuisses et/ou du volume des fesses, et notamment quantifiable. Au sens de la présente invention, on n'entend pas une perte de poids, mais un remodelage de la silhouette.

On entend par « diminuer les manifestations inesthétiques de la cellulite comme par exemple l'aspect peau d'orange et/ou la culotte de cheval », une diminution visuelle et mesurable des manifestations inesthétiques de la cellulite, par exemple par imagerie telle que la projection de franges à la surface de la peau (cette mesure est préférentiellement effectuée au niveau des hanches et/ou des fesses et/ou du ventre et/ou des cuisses, préférentiellement encore chez la femme), et/ou une diminution visuellement observable et quantifiable de l'aspect peau d'orange de la peau c'est-à-dire la diminution des capitons visibles et/ou amas graisseux superficiels du corps, notamment à long terme en évitant leur réapparition, et l'apparence plus lisse, plus homogène de la surface de la peau, préférentiellement au niveau des cuisses (en particulier du haut des cuisses) et/ou des hanches et/ou du ventre et/ou des fesses. On entend au sens de la présente invention par un « aspect peau d'orange » un aspect capitonné de la peau, non lisse, qui se manifeste par la présence de crevasses et capitons dus à une déformation des tissus sous-cutanés de la peau, de façon particulière de l'hypoderme, en raison notamment d'une augmentation de la taille des adipocytes.

On entend par « prévenir les manifestations inesthétiques de la cellulite comme par exemple l'aspect peau d'orange et/ou la culotte de cheval », empêcher l'apparition desdites manifestations.

On entend par « traiter les manifestations inesthétiques de la cellulite comme par exemple l'aspect peau d'orange et/ou la culotte de cheval », faire disparaitre lesdites manifestations.

Ainsi au sens de la présente invention, l'utilisation de l'extrait de *Lythrum salicaria* et/ou d'un des dérivés de l'acide gallique choisi dans le groupe constitué par la vescalagine, la castalagine et leur mélange, pour maintenir et/ou augmenter la lipolyse dans les adipocytes de la peau, pour induire un effet amincissant au niveau de la peau et/ou pour diminuer, prévenir et /ou traiter les manifestations inesthétiques de la cellulite comme par exemple l'aspect peau d'orange de la peau et/ou la culotte de cheval, est une utilisation cosmétique non thérapeutique qui n'est pas destinée au traitement de l'obésité ni au traitement de pathologie cutanée.

L'extrait selon l'invention peut être un extrait de tout ou partie de la plante choisi parmi la plante entière, les parties aériennes, la racine, les graines, les fleurs, les pétales, les sépales, les pétioles, les pédoncules floraux, les sommités fleuries, les feuilles, le fruit, la tige et/ou l'une quelconque de leur combinaison. Préférentiellement, l'extrait selon l'invention est un extrait des parties aériennes de la plante. Au sens de la présente invention, on entend par « parties aériennes » un mélange de feuilles, de fleurs, pédoncules floraux, et de la tige.

L'extrait de *Lythrum salicaria* selon l'invention est obtenu par tout procédé classique d'extraction de plantes connu de l'Homme du métier. La plante entière ou la partie de la plante concernée est ainsi séchée et/ou broyée avant extraction. L'extraction peut être réalisée par macération, par décoction à chaud, par broyage à l'aide d'un broyeur à billes, broyage au mortier, aux ultrasons, par broyage à l'aide d'un mixeur ou encore par extraction en conditions subcritiques ou supercritiques (dioxyde de carbone). Préférentiellement, l'extraction est réalisée par macération.

Dans un mode de réalisation préférentiel de l'invention, l'extrait de *Lythrum salicaria* est obtenu par macération d'au moins une partie de la plante, préférentiellement les parties aériennes, dans un solvant ou un mélange de solvants, de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol et/ou butylène glycol et/ou autres glycols, tel que xylitol etc.) en toutes proportions, en particulier de 100/0 à 0/100 (p/p). Plus préférentiellement, le solvant utilisé est constitué par de l'eau seulement. Encore plus préférentiellement le solvant utilisé est un mélange eau/alcool, en particulier eau/éthanol, de 15/85 à 85/15 (p/p), en particulier de 25/75 à 75/25 (p/p), plus particulièrement de 60/40 à 85/15 (p/p), en particulier de 75/25 (p/p).

En particulier, l'extrait est obtenu par extraction aqueuse. Au sens de la présente invention, on entend par « extrait obtenu par extraction aqueuse » tout extrait obtenu par extraction avec une solution aqueuse contenant plus de 60% en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de glycol et de façon particulière ne contenant pas d'alcool, plus particulièrement ne contenant que de l'eau.

Encore plus préférentiellement, l'extrait selon l'invention est un extrait de *Lythrum salicaria* obtenu par extraction avec une solution aqueuse contenant de l'éthanol, préférentiellement en une quantité de 15 % à 40 % en poids (p/p), encore préférentiellement de 25 % en poids (p/p) par rapport au poids total de la solution aqueuse.

L'extraction peut être réalisée durant une période allant de 1 heure à 24 heures, en particulier de 1 heure à 20 heures, préférentiellement de 2 heures à 24 heures, en particulier de 2 heures à 16 heures. Plus préférentiellement, l'extraction est réalisée durant une période de 2 heures. Dans un mode particulièrement avantageux de réalisation de l'invention, l'extraction est réalisée durant une période de 24 heures.

L'extraction peut être réalisée à une température comprise entre 0°C et 85 °C, préférentiellement entre 0 °C et 25 °C, plus préférentiellement entre 4 °C et 20 °C. Encore plus préférentiellement, l'extraction est réalisée à température ambiante, c'est-à-dire à 20 °C.

L'extrait peut être obtenu par extraction d'une quantité de 0,1% à 10 % en poids de matière sèche d'au moins une partie de la plante de *Lythrum salicaria* par rapport au poids total du mélange solvant/plante (p/p). Préférentiellement, l'extrait est obtenu par extraction d'une quantité de 1% à 10 % en poids, en particulier de 1 à 5% en poids, et avantageusement encore de 5% ou de 10% en poids de matière sèche d'au moins une partie de la plante, par rapport au poids total du mélange constitué du solvant, préférentiellement l'eau, et de la plante (p/p).

Dans un mode de réalisation préférentiel de l'invention, l'extrait est obtenu par macération durant une période de 2 heures à température ambiante, c'est-à-dire à 20°C, dans l'eau comme unique solvant, de 5% en poids des parties aériennes, c'est-à-dire du mélange de feuilles, de fleurs, leurs pédoncules, et de la tige de la plante Lythrum salicaria, par rapport au poids total de l'eau et des parties aériennes, tel que décrit dans l'exemple 1a).

Dans un autre mode de réalisation de l'invention, l'extrait est obtenu par macération durant une période de 1 heure à une température de 80°C, dans l'eau comme unique solvant, de 5% en poids des parties aériennes, c'est-à-dire du mélange de feuilles, de fleurs, leurs pédoncules, et de la tige, de la plante *Lythrum salicaria,* par rapport au poids total de l'eau et des parties aériennes, tel que décrit dans l'exemple 1b).

Dans encore un autre mode de réalisation de l'invention, l'extrait est obtenu par macération durant une période de 16 heures à une température de 4°C, dans l'eau comme unique solvant, de 5% en poids des parties aériennes, c'est-à-dire du mélange de feuilles, de fleurs, leurs pédoncules, et de la tige, de la plante *Lythrum salicaria,* par rapport au poids total de l'eau et des parties aériennes, tel que décrit dans l'exemple 1c).

Dans encore un autre mode de réalisation de l'invention, l'extrait est obtenu par macération durant une période de 16 heures à une température de 4°C, dans l'eau comme unique solvant, de 1% en poids des parties aériennes, c'est-à-dire du mélange feuilles, tige et fleurs, de la plante *Lythrum salicaria,* par rapport au poids total de l'eau et des parties aériennes, tel que décrit dans l'exemple 1d).

Dans un mode alternatif de réalisation de l'invention, l'extrait est obtenu par macération durant une période de 1 heure à une température de 80°C, dans l'eau comme unique solvant, de 5% en poids des feuilles de la plante *Lythrum salicaria,* par rapport au poids total de l'eau et des feuilles, tel que décrit dans l'exemple 1e).

Dans encore un autre mode de réalisation de l'invention, l'extrait selon l'invention est obtenu par macération dans un mélange eau/éthanol (75/25p/p) durant une période de 24 heures d'une quantité de 10 % en poids des parties aériennes, c'est-à-dire du mélange de feuilles, de fleurs, leurs pédoncules, et de la tige, par rapport au poids total du mélange de solvant et des parties aériennes de la plante, dans les conditions décrites selon l'exemple 1g).

L'extrait brut obtenu et utilisé selon l'invention est de préférence ensuite centrifugé et/ou filtré et/ou distillé pour récupérer la fraction soluble, préférentiellement la fraction hydrosoluble. Préférentiellement, le surnageant obtenu est ensuite filtré, avantageusement à un seuil de coupure de 0,45 µm. Des étapes supplémentaires de décoloration et/ou désodorisation peuvent être effectuées sur l'extrait à n'importe quel stade de l'extraction et selon les techniques connues par l'Homme du métier.

L'extrait peut être ensuite purifié, par différentes méthodes de purification connues de l'homme du métier, par exemple par chromatographie. Préférentiellement, l'extrait selon l'invention est purifié dans une première étape par extraction sur phase solide (SPE), en particulier de silice greffée C18. L'extrait est déposé en solution hydroalcoolique contenant par exemple 20 % de méthanol et 0,5 % d'acide formique. L'élution est réalisée avec ce même solvant. L'extrait obtenu peut être ensuite concentré par évaporation puis lyophilisé. L'extrait obtenu peut être ensuite purifié, par exemple dans une deuxième étape, par chromatographie liquide haute pression (HPLC) semi-préparative, préférentiellement sur colonne Synergi Fusion

(Phenomenex) de dimensions 250x10mm. L'élution est effectuée par exemple par un gradient acide formique 0,5 % et méthanol.

L'extrait de *Lythrum salicaria* purifié selon l'invention, en particulier ainsi obtenu, comprend de la vescalagine et/ou de la castalagine, en une quantité totale en molécules d'au moins 0,25 %, préférentiellement d'au moins 0,5 %, encore préférentiellement d'au moins 1 % en poids par rapport au poids total de l'extrait purifié.

Une étape supplémentaire d'ajout de maltodextrine peut être mise en oeuvre, par exemple après la purification de l'extrait. Avantageusement une quantité d'au moins 20 % en poids par rapport au poids total de l'extrait et de la maltodextrine, préférentiellement d'au moins 50 %, encore préférentiellement d'au moins 70 % en poids de maltodextrine, est alors ajoutée à l'extrait. L'extrait peut être ensuite concentré par évaporation du solvant ou séché par exemple par lyophilisation ou par atomisation. Préférentiellement, l'extrait est séché par atomisation.

Dans un mode préférentiel de réalisation de l'invention, l'extrait est séché par atomisation après ajout de maltodextrine dans la solution. Avantageusement, la quantité en poids de maltodextrine ajoutée à l'extrait avant l'étape de séchage par atomisation est telle que l'extrait comprend 1 % en poids de vescalagine et/ou castalagine par rapport au poids total de la poudre ainsi obtenue.

Dans un mode particulier de réalisation de l'invention, notamment pour son utilisation en dermatologie, l'extrait de *Lythrum salicaria* obtenu est stérilisé.

Selon l'invention, l'extrait de *Lythrum salicaria* et/ou un des dérivés de l'acide gallique choisi dans le groupe constitué par la vescalagine, la castalagine et leur mélange, peut être utilisé seul sous forme d'ingrédient actif et/ou dans une composition cosmétique et/ou dermatologique destinée à une application par voie topique, préférentiellement sur la peau et/ou les muqueuses.

Lorsqu'il est utilisé seul sous forme d'ingrédient actif, l'extrait et/ou un des dérivés de l'acide gallique choisi dans le groupe constitué par la vescalagine, la castalagine et leur mélange, plus préférentiellement la castalagine, est préférentiellement soluble et/ou dilué dans un solvant notamment polaire, tel que l'eau, un alcool, un polyol, un glycol, tel que le pentylène glycol et/ou le butylène glycol et/ou l'hexylène glycol et/ou le caprylyl glycol, ou un de leurs mélanges, préférentiellement un mélange hydroglycolique, encore préférentiellement contenant un glycol choisi parmi l'hexylène glycol, le caprylyl glycol et leurs mélanges.

Avantageusement l'extrait obtenu et/ou un des dérivés de l'acide gallique choisi dans le groupe constitué par la vescalagine, la castalagine et leur mélange, plus préférentiellement la castalagine, est dilué et/ou soluble dans une solution aqueuse contenant de l'hexylène glycol, en particulier contenant entre 0,1 et 10 % en poids de l'hexylène glycol, préférentiellement entre 0,5 et 5 % en poids d'hexylène glycol, par rapport au poids total la solution aqueuse.

Avantageusement l'extrait obtenu et/ou un des dérivés de l'acide gallique choisi dans le groupe constitué par la vescalagine, la castalagine et leur mélange, plus préférentiellement la castalagine, est dilué et/ou soluble dans une solution aqueuse contenant du caprylyl glycol, en particulier contenant entre 0,01 et 5 % en poids de caprylyl glycol, préférentiellement entre 0,1 et 1 % en poids de caprylyl glycol, par rapport au poids total la solution aqueuse.

De façon particulière, la solution aqueuse dans laquelle est solubilisée l'extrait de *Lythrum salicaria* selon l'invention et/ou un des dérivés de l'acide gallique choisi dans le groupe constitué par la vescalagine, la castalagine et leur mélange, plus préférentiellement la castalagine, comprend de la gomme xanthane, en particulier entre 0,01 et 5 % en poids de gomme xanthane par rapport au poids total la solution aqueuse, plus particulièrement entre 0,1 et 1 % en poids de gomme xanthane par rapport au poids total de la solution aqueuse.

De façon particulièrement avantageuse, la solution dans laquelle est solubilisé l'extrait de *Lythrum salicaria* selon l'invention et/ou un des dérivés de l'acide gallique choisi dans le groupe constitué par la vescalagine, la castalagine et leur mélange, plus préférentiellement la castalagine, comprend de l'hexylène glycol, du caprylyl glycol et de la gomme de xanthane, en particulier dans les proportions indiquées dans l'exemple 4. L'extrait de *Lythrum salicaria* et/ou un des dérivés de l'acide gallique choisi dans le groupe constitué par la vescalagine, la castalagine et leur mélange, plus préférentiellement la castalagine, peut aussi être utilisé dans une composition cosmétique et/ou dermatologique. Ainsi, la composition comprend au moins un excipient cosmétiquement et/ou dermatologiquement acceptable.

Les termes « excipient cosmétiquement et/ou dermatologiquement acceptable », utilisés ici, signifient que la composition ou les composants de celle-ci sont adaptés à l'utilisation en contact avec la peau humaine sans toxicité, incompatibilité, instabilité, réponse allergique, ou leurs équivalents, indue.

Dans un mode de réalisation de l'invention, l'extrait de *Lythrum salicaria* est présent dans la composition cosmétique et/ou dermatologique à une concentration de 1×10⁻⁴ % à 10%, préférentiellement de 1×10⁻⁴ % à 5 %, et encore préférentiellement de 1×10⁻³ % à 3% en poids, par rapport au poids total de la composition.

La composition cosmétique peut donc comprendre un des dérivés de l'acide gallique choisi dans le groupe constitué par la vescalagine, la castalagine et leur mélange, plus préférentiellement la castalagine, à une concentration finale totale en molécules de 2,5×10⁻⁷ à 0,1 %, préférentiellement de 5×10⁻⁷ à 0,05 %, encore préférentiellement de 1×10⁻⁵ à 0,03 % en poids, par rapport au poids total de la composition.

Avantageusement, le ou lesdits excipients sont choisis parmi au moins un des groupes constitués par les conservateurs, les émollients, les émulsifiants, les tensioactifs, les hydratants, les épaississants, les agents de texture, les agents filmogènes, les pigments, les stabilisants, les solubilisants, les colorants, les parfums.

Avantageusement encore, le ou les excipients sont choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les gommes de xanthane, les cires naturelles et synthétiques, les huiles végétales, les triglycérides, les insaponifiables, les phytostérols, les esters végétaux, les silicones et ses dérivés, les hydrolysats de protéines, l'huile de Jojoba et ses dérivés, les esters lipo/hydrosolubles, les betaines, les aminoxides, les extraits de plantes, les esters de Saccharose, les dioxydes de Titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparaben, l'éthylparaben, le propylparaben, le butylparaben, le butylène glycol, le caprylyl glycol, les tocophérols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de coeur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

La composition cosmétique et/ou dermatologique de l'invention peut être choisie parmi une solution, aqueuse ou huileuse, une crème ou un gel aqueux ou un gel huileux, notamment un gel douche, un shampoing, un lait, un lait pour le corps, une émulsion, une microémulsion ou une nanoémulsion, notamment huile-dans-eau ou eau-dans-huile ou multiple ou siliconée, un masque, un sérum, une lotion, un savon liquide, un pain dermatologique, une pommade, une mousse, un patch.

Préférentiellement, la composition cosmétique selon l'invention est une crème ou un sérum. Dans un mode alternatif de réalisation de l'invention, la composition est un lait pour le corps.

De façon avantageuse, la composition cosmétique et/ou dermatologique est destinée à être appliquée sur au moins une zone de peau et/ou de muqueuse choisie parmi le ventre, les bras, les avant-bras, les genoux, le buste, les cuisses, les hanches, les fesses, le cou, la taille et/ou le visage, et préférentiellement le corps, en particulier sur une zone de peau comprenant de la cellulite et/ou ses manifestations inesthétiques telles que la peau d'orange ou la culotte de cheval et/ou sur une zone de peau manquant de fermeté en raison de variations physiologiques non pathologiques telles qu'un changement de régime alimentaire, les variations hormonales, en particulier durant la puberté, la grossesse, la ménopause mais aussi une mauvaise alimentation, la sédentarité et plus particulièrement chez la femme. Dans un mode alternatif de réalisation de l'invention, la composition cosmétique et/ou dermatologique est appliquée, en particulier par massage avec des mouvements circulaires, sur des parties spécifiques du corps choisies parmi le ventre, les bras, les cuisses, les hanches, les fesses et/ou la taille, préférentiellement les cuisses et les hanches.

En outre, la composition cosmétique de la présente invention peut contenir un ou plusieurs autres ingrédients actifs cosmétiques, conduisant à un effet complémentaire et/ou un effet de synergie avec l'extrait de *Lythrum salicaria* selon l'invention et/ou un des dérivés de l'acide gallique choisi dans le groupe constitué par la vescalagine, la castalagine et leur mélange, plus préférentiellement la castalagine,.

Il peut s'agir par exemple d'agents tenseurs pour un effet de synergie avec l'extrait selon l'invention. Avantageusement, il s'agit d'ingrédients actifs augmentant l'expression génique et/ou protéique du collagène et/ou empêchant la dégradation dudit collagène tels que le rétinol, la vitamine C, un extrait de Quassia amara ou un extrait de Davilla rugosa commercialisé sous le nom de Collguard^{™} par BASF Beauty Care Solutions, ou des agents actifs stimulant la synthèse des macromolécules du derme ou empêchant leur dégradation comme :
- un agent de protection du facteur de croissance des fibroblastes (FGF2) de la matrice extracellulaire contre sa dégradation et/ou sa dénaturation, notamment un extrait d'Hibiscus abelmoscus tel que décrit dans la demande de brevet au nom de la Demanderesse déposée sous le numéro FR0654316;
- un agent de stimulation de croissance des fibroblastes par exemple un extrait de soja fermenté contenant des peptides, connu sous le nom de Phytokine^{™} commercialisé par la Demanderesse et également décrit dans la demande de brevet EP1119344 ;
- et/ou encore un agent stimulant la synthèse de fibronectine, en particulier un extrait de maïs, un tel extrait étant notamment commercialisé par la Demanderesse sous le nom Deliner^{™} ou les produits commercialisés sous les noms de Matrixyl^{™} (le palmitoyl pentapeptide), Matrixyl 3000^{™} et Regestril^{™} par la société Sederma ;
- un agent stimulant la synthèse de laminine, en particulier un extrait de malt modifié par biotechnologie, un tel extrait étant notamment commercialisé par la Demanderesse sous le nom Basaline^{™} ;
- un agent stimulant l'expression et/ou l'activité de la Hyaluronane synthase 2 (HAS2) tels que les extraits végétaux décrits dans la demande de brevet FR2 893 252 et en particulier un extrait aqueux de Galanga (Alpinia galanga) ;
- un agent stimulant la synthèse de lysyl oxydase like (LOXL) tel que un extrait de Geophila cordifolia et ceux décrits dans la demande de brevet FR2855968, et en particulier un extrait d'aneth ;
- un agent stimulant la synthèse d'ATP intracellulaire, notamment un extrait d'algue Laminaria digitata ;
- un actif stimulant la synthèse des glycosaminoglycanes, tels que le produit de fermentation du lait ;
- un actif inhibiteur des métalloprotéinases (MMP) telles que plus particulièrement les MMP 1, 2, 3, 9 tel que les rétinoïdes et dérivés, les oligopeptides et les lipopeptides, les lipoaminoacides, l'extrait de malt commercialisé par BASF Beauty Care Solutions France sous la dénomination commerciale Collalift^{™}, l'extrait hydrolysé de pomme de terre commercialisé sous le nom Extracellium^{™} par BASF Beauty Care solutions France SAS; le lycopène ; les isoflavones, la quercetine, le kaempferol, l'apigenine.

Il peut aussi s'agir d'agents stimulant la prolifération des kératinocytes, notamment les rétinoïdes tels que le rétinol et ses esters, dont le palmitate de rétinyle et le phloroglucinol et/ou d'agents stimulant la différenciation des kératinocytes comprennent par exemple les minéraux tels que le calcium et les lignanes tels que le sécoisolaricirésinol ainsi que l'extrait d'Achillea millefollium commercialisé sous le nom de Neurobiox^{™} par BASF Beauty Care Solutions France.

Les agents tenseurs utilisables dans l'invention peuvent être aussi choisis parmi les polymères synthétiques, tels que les latex de polyuréthanne ou les latex acryliques, les polymères d'origine naturelle, notamment les polyholosides sous forme d'amidon ou sous forme de carraghénanes, alginates, agars, gellanes, polymères cellulosiques et pectines, les protéines et hydrolysats de protéines végétales de soja, les silicates mixtes, les microparticules de cire, les particules colloïdales de charge inorganique choisies par exemple parmi la silice, les composites silice-alumine ; ainsi que leurs mélanges. Il peut enfin s'agir d'ingrédients cosmétiques et/ou dermatologiques comme par exemple des agents antimicrobiens, agents anti-radicalaires, agents apaisants, calmants ou relaxants, agents agissant sur la microcirculation pour augmenter l'éclat du teint, en particulier du visage, agents cicatrisants.

Parmi les agents antimicrobiens pouvant être associés à l'ingrédient actif de l'invention dans la présente invention, on peut citer le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octoxyglycérine, l'octanoylglycine, le caprylyl glycol, l'acide 10-hydroxy-2-décanoïque, le farnesol, les phytosphingosines et leurs mélanges. Les agents anti-radicalaires peuvent être la vitamine C et ses dérivés dont le glucoside d'ascorbyle, les phénols et polyphénols, en particulier les tannins, ; l'épigallocatéchine et les extraits naturels en contenant, en particulier les extraits de thé vert, les anthocyanes, les acides phénols, les stilbènes, des actifs piégeurs de composés aromatiques mono- ou polycycliques les tannins et les dérivés indoles et/ou des actifs piégeurs de métaux lourds tels que l'EDTA, des actifs anti-radicalaires tels que la vitamine E et ses dérivés tels que l'acétate de tocophéryle, les bioflavonoïdes, la coenzyme Q10 ou ubiquinone.

La composition cosmétique de la présente invention peut aussi contenir un ou plusieurs agents anti-radicalaires et/ou anti-oxydants tel qu'un extrait de racine de Rhodiola crenulata commercialisé sous le nom de Rhodiomax^{™} par la demanderesse.

Comme agents apaisants entrant dans la composition de l'invention, on peut utiliser les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de *Pueraria lobata* commercialisé sous le nom Inhipase^{™} par la demanderesse, les extraits de *Theobroma cacao.* Les ingrédients actifs agissant sur la microcirculation, vasoprotecteurs ou vasodilatateurs, peuvent être choisis parmi les flavonoïdes, les ruscogénines, les nicotinates, les huiles essentielles.

Enfin, la composition peut aussi contenir en combinaison avec l'extrait selon l'invention et/ou un des dérivés de l'acide gallique choisi dans le groupe constitué par la vescalagine, la castalagine et leur mélange, plus préférentiellement la castalagine, et/ou les ingrédients actifs cités ci-dessus des ingrédients drainants et/ou amincissants et/ou qui préviennent et/ou traitent les manifestations inesthétiques de la cellulite comme par exemple l'aspect peau d'orange tels que la caféine et/ou la théophylline, les polyphénols de *Theobroma cacao,* un hydrolysat d'algue rouge commercialisée sous le nom de SlimExcess^{™} par la demanderesse, un extrait de racine de *Coleus forskohlii*, un extrait de *Ginkgo biloba,* de façon particulière un extrait de feuille de *Ginkgo biloba,* un extrait d'écorce de *Cecropia obtusa*, un extrait de laitue de mer (*Ulva lactuca*), un extrait de feuille de *Peumus boldus*, un extrait de *Smallanthus sonchifolius* aussi appelée yacon, ou encore une combinaison d'extraits hydrolysés de fruit de *Coriandrum sativum* et de fruit de *Citrus aurantium dulcis,* une combinaison d'extraits hydrolysés de *Celosia cristata* et de *Prunella vulgaris,* associés ou non à des ingrédients actifs agissant sur la microcirculation, vasoprotecteurs ou vasodilatateurs choisis parmi l'escine, les flavonoïdes, les ruscogénines, les nicotinates, les huiles essentielles, ou un actif cosmétique stimulant la microcirculation cutanée notamment par un effet froid tels que les extraits de menthe et le menthol.

En outre, la présente invention a pour objet un procédé de soin non thérapeutique comprenant l'application par voie topique, sur au moins une zone de peau et/ou de muqueuse saine, de l'extrait de *Lythrum salicaria* selon l'invention ou d'une composition cosmétique comprenant un tel extrait selon l'invention, pour maintenir et/ou augmenter l'expression de collagène pour maintenir et/ augmenter la fermeté de la peau et/ou des muqueuses, en particulier humain.

La présente invention a également pour objet un procédé de soin non thérapeutique comprenant l'application par voie topique, sur au moins une zone de peau et/ou de muqueuse saine, de l'extrait de *Lythrum salicaria* selon l'invention et/ou d'un des dérivés de l'acide gallique choisi dans le groupe constitué par la vescalagine, la castalagine et leur mélange, plus préférentiellement la castalagine, ou d'une composition cosmétique comprenant un tel extrait selon l'invention et/ou un des dérivés de l'acide gallique choisi dans le groupe constitué par la vescalagine, la castalagine et leur mélange, plus préférentiellement la castalagine, pour maintenir et/ou augmenter la lipolyse dans les adipocytes de la peau pour induire un effet amincissant au niveau de la peau et/ou diminuer, prévenir et /ou traiter les manifestations inesthétiques de la cellulite comme par exemple l'aspect peau d'orange de la peau et/ou la culotte de cheval et/ou pour la prévention et/ou le traitement de la cellulite. Préférentiellement, le collagène est le collagène de type I.

Avantageusement, le procédé de soin non thérapeutique comprend l'application par voie topique, sur au moins une zone de peau et/ou de muqueuse saine, de l'extrait de *Lythrum salicaria* comprenant au moins 0,25 %, préférentiellement au moins 0,5 %, encore préférentiellement au moins 1 % en poids de vescalagine et/ou de castalagine par rapport au poids total de l'extrait de *Lythrum salicaria.* Dans un mode de réalisation de l'invention, le procédé de soin non thérapeutique comprend l'application par voie topique, avantageusement par massage avec des mouvements circulaires, de l'extrait de *Lythrum salicaria* selon l'invention et/ou d'un des dérivés de l'acide gallique choisi dans le groupe constitué par la vescalagine, la castalagine et leur mélange, sous la forme de la composition cosmétique selon l'invention. Selon un mode de réalisation de l'invention, le procédé de soin non thérapeutique est appliqué sur au moins une zone de peau et/ou de muqueuse choisie parmi le ventre, les bras, les avant-bras, les genoux, le buste, les cuisses, les hanches, les fesses, le cou, la taille et/ou le visage, et préférentiellement le corps, en particulier, sur une zone de peau comprenant de la cellulite et/ou ses manifestations inesthétiques telles que la peau d'orange ou la culotte de cheval et/ou sur une zone de peau manquant de fermeté en raison de variations physiologiques non pathologiques telles qu'un changement de régime alimentaire, les variations hormonales, en particulier durant la puberté, la grossesse, la ménopause mais aussi une mauvaise alimentation, la sédentarité et plus particulièrement chez la femme.

L'invention a également pour objet l'extrait de la plante *Lythrum salicaria,* préférentiellement obtenu par extraction aqueuse et préférentiellement encore par extraction avec une solution aqueuse ne contenant que de l'eau ou contenant au moins 70 % en poids d'eau par rapport au poids total de la solution aqueuse, avantageusement obtenu par extraction avec une solution aqueuse contenant 25 % en poids d'alcool, en particulier d'éthanol par rapport au poids total de la solution aqueuse, et/ou un des dérivés de l'acide gallique choisi dans le groupe constitué par la vescalagine, la castalagine et leur mélange, plus préférentiellement la castalagine, seul ou dans une composition dermatologique le comprenant avec un excipient dermatologiquement acceptable, pour son utilisation, avantageusement par voie topique, pour le traitement et/ou la prévention des pathologies de la peau et/ou des muqueuses impliquant une perte d'expression de collagène, préférentiellement du collagène de type I, encore préférentiellement humain, et/ou une perte de fermeté pathologique, telles que les télangiectasies et/ou impliquant un dysfonctionnement dans la lipolyse au niveau des tissus adipeux, impliqué notamment dans des maladies telles que l'obésité. Avantageusement, l'extrait de *Lythrum salicaria* est un extrait des parties aériennes de la plante, c'est-à-dire un mélange de feuilles, fleurs, pédoncules floraux, et tige de la plante *Lythrum salicaria.* Avantageusement encore, l'extrait de *Lythrum salicaria* comprend au moins 0,25 %, préférentiellement au moins 0,5 %, encore préférentiellement au moins 1 % en poids d'acide gallique et/ou d'un des dérivés de l'acide gallique, en particulier de vescalagine et/ou de castalagine, par rapport au poids total de l'extrait, en particulier purifié.

L'invention a également pour objet l'extrait de la plante *Lythrum salicaria,* préférentiellement obtenu par extraction aqueuse et préférentiellement encore par extraction avec une solution aqueuse ne contenant que de l'eau ou contenant au moins 70 % en poids d'eau par rapport au poids total de la solution aqueuse, avantageusement obtenu par extraction avec une solution aqueuse contenant 25 % en poids d'alcool, en particulier d'éthanol, et/ou un des dérivés de l'acide gallique choisi dans le groupe constitué par la vescalagine, la castalagine et leur mélange, seul ou dans une composition dermatologique le comprenant avec un excipient dermatologiquement acceptable, pour son utilisation, par voie topique, pour le traitement et/ou la prévention des pathologies de la peau et/ou des muqueuses impliquant une diminution de la lipolyse.

Des exemples faisant référence à la description de l'invention sont présentés ci-après. Ces exemples sont donnés à titre d'illustration et ne sauraient limiter en aucun cas la portée de l'invention. Chacun des exemples a une portée générale.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention.

### Exemple 1 : Différents modes de réalisation de l'invention pour l'obtention d'un extrait de Lythrum salicaria selon l'invention.

### a) Obtention d'un extrait des parties aériennes de Lythrum salicaria selon l'invention

Cinq pour cent de parties aériennes, c'est-à-dire d'un mélange de feuilles, de fleurs, pédoncules floraux et tige, en poids de matière sèche par rapport au poids total du mélange constitué de l'eau et des parties aériennes de la plante *Lythrum salicaria* (p/p), ont été mis à macérer dans l'eau, l'eau étant ici l'unique solvant, durant une période de 2 heures à température ambiante, c'est-à-dire à 20 °C. Après macération, l'extrait brut obtenu a été centrifugé 10 min (8000 tour par min) puis le surnageant récupéré et filtré (seuil de coupure de 0,45 µm). L'extrait de parties aériennes à 5 % (p/p) a ensuite été testé sur l'augmentation de l'expression protéique du collagène de type I dans les conditions présentées dans l'exemple 2 ci-après.

### b) Obtention d'un extrait des parties aériennes de Lythrum salicaria selon l'invention.

Cinq pour cent de parties aériennes, c'est-à-dire d'un mélange de feuilles, de fleurs, pédoncules floraux et tige, en poids de matière sèche par rapport au poids total du mélange constitué de l'eau et des parties aériennes de la plante *Lythrum salicaria* (p/p) ont été mis à macérer dans l'eau à une température de 80°C durant une période de 1 heure. L'extrait brut a ensuite été centrifugé pendant 10 min (8000 tour par min) puis le surnageant filtré (0,45 µm).

### c) Obtention d'un extrait des parties aériennes de Lythrum salicaria selon l'invention.

Cinq pour cent de parties aériennes, c'est-à-dire d'un mélange de feuilles, de fleurs, pédoncules floraux et tige, en poids de matière sèche par rapport au poids total du mélange constitué de l'eau et des parties aériennes de la plante *Lythrum salicaria* (p/p) ont été mis à macérer à une température de 4°C, durant une période de 16 heures, dans de l'eau. L'extrait brut a ensuite été centrifugé pendant 10 min (8000 tour par min) puis le surnageant filtré (0,45 µm).

### d) Obtention d'un extrait des parties aériennes de Lythrum salicaria selon l'invention.

Un pour cent de parties aériennes en poids de matière sèche par rapport au poids total du mélange constitué de l'eau et des parties aériennes de la plante *Lythrum salicaria* (p/p) ont été mis à macérer à une température de 4 °C durant une période de 16 heures dans de l'eau. L'extrait brut a ensuite été centrifugé pendant 10 min (8000 tour par min) puis le surnageant filtré (0,45 µm).

### e) Obtention d'un extrait de feuilles de Lythrum salicaria selon l'invention.

Cinq pour cent de feuilles en poids de matière sèche par rapport au poids total du mélange constitué du solvant et des feuilles (p/p) de la plante *Lythrum salicaria* ont été mis à macérer dans de l'eau comme unique solvant durant une période de 1 heure à une température de 80°C. L'extrait brut a ensuite été centrifugé pendant 10 min (8000 tour par min) puis le surnageant filtré (0,45 µm).

### f) Obtention d'un extrait de feuilles de Lythrum salicaria selon l'invention

Une quantité de 5% de feuilles de *Lythrum salicaria* en poids de matière sèche par rapport au poids total du mélange constitué de l'eau et de la plante *Lythrum salicaria* a été mise à macérer à température ambiante, ici 20 °C, durant une période de 2 heures, dans un mélange eau/butylène glycol (75/25 ; p/p). L'extrait brut a ensuite été centrifugé pendant 10 min (8000 tours par min) pour éliminer la fraction insoluble puis le surnageant filtré (0,45 µm).

### g) Obtention d'un extrait de Lythrum salicaria comprenant de la vescalagine et/ou de la castalagine selon l'invention

Une quantité de 10 % des parties aériennes, c'est-à-dire du mélange de feuilles, de fleurs, leurs pédoncules, et de la tige de *Lythrum salicaria,* en poids de matière sèche par rapport au poids total du mélange constitué du solvant et des parties aériennes de la plante, a été mise à macérer sous agitation à température ambiante, ici 20 °C, durant une période de 24 heures, dans un mélange eau/éthanol (75/25 ; p/p).

L'extrait a ensuite été purifié dans une première étape par extraction sur phase solide (SPE) de silice greffée C18. L'extrait a été déposé en solution hydroalcoolique contenant 20 % de méthanol et 0,5 % d'acide formique. L'élution a été réalisée avec ce même solvant. L'extrait obtenu a ainsi été concentré par évaporation puis lyophilisé. L'extrait obtenu a ensuite été purifié dans une deuxième étape par chromatographie liquide haute pression (HPLC) semi-préparative sur colonne Synergi Fusion (Phenomenex) de dimensions 250x10mm. L'élution a été effectuée par un gradient acide formique 0,5 % et méthanol.

L'extrait purifié ainsi obtenu comprend de la vescalagine et/ou de la castalagine, en une quantité totale en molécules de 1 % en poids par rapport au poids total de l'extrait purifié.

De la maltodextrine a été ajoutée à l'extrait puis l'extrait a été concentré par évaporation puis lyophilisé.

### Exemple 2 : Mise en évidence de l'effet d'un extrait de Lythrum salicaria selon l'invention sur l'expression protéique du collagène de type I.

### Protocole :

Les conditions expérimentales de mise en évidence de l'augmentation de l'expression protéique du collagène de type I sont celles exposées dans l'exemple 1 de la demande internationale publiée sous le numéro WO2012/175454.

Des fibroblastes humains normaux, c'est-à-dire non pathologiques, obtenus à partir de biopsies abdominales d'un donneur sain ont été ensemencés en plaque 96 puits et cultivés dans un milieu défini (FGM) jusqu'à 100% de confluence, ce qui est obtenu après 3 jours de culture.

L'extrait de *Lythrum salicaria* selon l'invention préparé selon l'exemple 1a de la présente invention a été ajouté à une concentration finale de 1 % (p/p) dans le milieu de culture. Le même milieu de culture sans ajout d'extrait selon l'invention a été utilisé comme contrôle (témoin non traité).

Après 48 heures de culture post confluence à 37°C, le milieu de culture a été prélevé et éliminé. Une étape de lyse des cellules a été effectuée puis le lysat a été prélevé et analysé. Un dosage d'ADN a été réalisé sur les lysats de façon à exprimer l'expression protéique du collagène par cellule.

L'ADN double brin a été dosé par la méthode au bisbenzimide (Invitrogen, Quant-iT^{™} PicoGreen ^{®} dsDNA). La concentration en ADN est proportionnelle au nombre de cellules viables et permet de rationaliser la fluorescence lue par un nombre de cellules.

Le dosage du collagène de type I a été effectué par technique immunochimique comme suit :
Un anticorps anti-collagène de type I a été incubé durant 30 minutes avec le lysat cellulaire. Après rinçage au PBS, l'anticorps secondaire couplé à l'europium (Perkin Elmer) a été ajouté. Une solution de révélation a été ajoutée et la fluorescence a été mesurée en utilisant un lecteur multiplaque EnVision (Perkin Elmer).

Pour chaque condition, la fluorescence en temps retardée (TRF) a été mesurée dans chaque puits et rationalisée par la quantité d'ADN dosée dans le puits. Le ratio (fluorescence/concentration d'ADN) a été calculé.

Pour chaque condition, les résultats sont exprimés par le pourcentage de la moyenne des expressions protéiques par rapport aux expressions protéiques mesurées dans le contrôle (témoin non traité) (n=6).

### Résultats :

| | Moyenne | Ecart-type |
|---|---|---|
| Contrôle | 100 | 1 |
| Extrait 1a) de *Lythrum salicaria* 1 % (p/p) | 196,6 | 14,5 |

### Conclusion :

L'extrait de *Lythrum salicaria* selon l'invention a induit une augmentation de l'expression protéique du collagène de type I dans des fibroblastes humains cultivés *in vitro.* L'extrait selon l'invention peut donc être utilisé pour augmenter la fermeté de la peau et/ou des muqueuses.

### Exemple 3 : Effet de différents extraits de Lythrum salicaria selon l'invention sur l'augmentation de la lipolyse des adipocytes de la peau.

### Exemple 3a) Extrait de Lythrum salicaria selon l'exemple 1a)

### Protocole :

Des adipocytes ont été obtenus par différenciation de préadipocytes humains comme suit : les préadipocytes humain normaux, c'est-à-dire non pathologiques, non cancéreux, ont été ensemencés dans un milieu de croissance enrichi en facteurs de croissance (PAGS 7252 Cliniscience) et sérum de veau foetal (FBS). Après 3 jours de culture, le milieu de culture a été remplacé par un milieu de différentiation (PADM 7221 Cliniscience) contenant les facteurs de croissance et le FBS. Après 2 jours de culture, le milieu de différentiation a été remplacé par un milieu standard contenant 1% de FBS. Ensuite, les adipocytes formés ont été incubés durant une période de 16 heures en présence de l'extrait de *Lythrum salicaria* selon l'exemple 1a) à une concentration finale de 1% en poids par rapport au poids total du milieu de culture final et de l'extrait.

La lipolyse a été évaluée par quantification du glycérol libéré dans le milieu de culture. La quantification du glycérol a été obtenue par méthode enzymatique (kit Libios K-GCROL) et mesure de la densité optique à 340 nm. Les résultats sont exprimés en pourcentage par rapport au contrôle (milieu de culture sans extrait de *L. salicaria*) (Base 100). Les résultats présentés sont la moyenne de 3 essais (n=3).

### Résultats :

| | Moyenne | Ecart-type |
|---|---|---|
| Contrôle | 100 | 20,2 |
| Extrait 1a) de *Lythrum salicaria* 1 % (p/p) | 210,3 | 31,8 |

### Conclusion :

Les résultats ont montré une augmentation de la lipolyse dans les adipocytes cultivés en présence de l'extrait de *Lythrum salicaria,* montrant la capacité de l'extrait à induire un effet amincissant et diminuer les manifestations inesthétiques de la cellulite comme par exemple l'aspect peau d'orange et/ou la culotte de cheval.

### Exemple 3b) Extrait de Lythrum salicaria comprenant de la vescalagine et de castalagine selon l'exemple 1g).

### Protocole :

Des adipocytes ont été obtenus par différenciation de préadipocytes murins 3T3-L1 comme suit : les préadipocytes murins ont été ensemencés dans un milieu de croissance enrichi en sérum de veau néo-natal. Après 3 jours de culture, le milieu de culture a été remplacé par un milieu de différentiation contenant les facteurs de différenciation Iso-Butyl-Methyl-Xanthine (IBMX) et Dexamethasone, et du sérum de veau foetal (SVF). Après 2 jours de culture, le milieu de différentiation a été remplacé par un milieu standard contenant 10% de SVF et incubés durant 8 jours. Ensuite, les adipocytes formés ont été incubés durant une période de 16 heures en présence de l'extrait de Lythrum salicaria selon l'exemple 1g) à une concentration finale de 2,5×10⁻³ % en poids par rapport au poids total du milieu de culture final et de l'extrait.

La lipolyse a été évaluée par quantification du glycérol libéré dans le milieu de culture. La quantification du glycérol a été obtenue par méthode enzymatique (kit Libios K-GCROL) et mesure de la densité optique à 340 nm.

Les résultats sont exprimés en pourcentage par rapport au contrôle (milieu de culture sans extrait de *L. salicaria*) (Base 100). Les résultats présentés sont la moyenne d'un essai en "quadruplet" (n=4).

### Résultats :

| | **Moyenne** | **Ecart-type** |
|---|---|---|
| Contrôle | 100 | 12 |
| Extrait 1g) de *Lythrum salicaria* 2,5×10⁻³ % (p/p) | 151 | 4 |

### Conclusion :

Les résultats ont montré une augmentation d'au moins 35 % de la lipolyse dans les adipocytes murins cultivés en présence de l'extrait de *Lythrum salicaria* comprenant de la vescalagine et de la castalagine, montrant la capacité de l'extrait à induire un effet amincissant et diminuer les manifestations inesthétiques de la cellulite comme par exemple l'aspect peau d'orange et/ou la culotte de cheval.

### Exemple 4 : Exemple d'ingrédients cosmétiques et/ou dermatologiques contenant l'extrait de Lythrum salicaria selon l'invention.

On procède selon les méthodes connues de l'homme de l'art pour mélanger ensemble les différents ingrédients selon la présente invention. Les pourcentages exprimés sont des pourcentages en poids par rapport au poids total de la composition.

### a) L'extrait de Lythrum salicaria selon l'invention est celui obtenu selon l'exemple 1a.

| | |
|---|---|
| Extrait de *Lythrum salicaria* (Ex. 1a)) | 5 % |
| Hexylène glycol | 0,5 % |
| Caprylyl glycol | 1 % |
| Gomme de xanthane | 0,5 % |
| Eau | Qsp 100 |

### b) L'extrait de Lythrum salicaria selon l'invention est celui obtenu selon l'exemple 1b.

| | |
|---|---|
| Extrait de *Lythrum salicaria* (Ex. 1b)) | 5 % |
| Hexylène glycol | 0,5 % |
| Caprylyl glycol | 1 % |
| Gomme de xanthane | 0,5 % |
| Eau | Qsp 100 |

### c) L'extrait de Lythrum salicaria selon l'invention est celui obtenu selon l'exemple 1g).

| | |
|---|---|
| Extrait de *Lythrum salicaria* (Ex. 1g)) | 1-5 % |
| Maltodextrine | 80 % |
| Eau | Qsp 100 |

### Exemple 5 : Exemple de compositions cosmétiques contenant l'extrait selon l'invention.

Les compositions ci-après sont préparées selon des méthodes connues de l'homme du métier, en particulier s'agissant des différentes phases à mélanger entre elles.

### 5a) Composition cosmétique contenant un extrait de Lythrum salicaria selon l'invention

*L'ingrédient cosmétique est préparé selon l'exemple 4a ou 4c précédent. Les quantités indiquées sont en pourcentage en poids par rapport au poids total de la composition.

| | |
|---|---|
| Ingrédient cosmétique* | 3,00 |
| EDTA | 0,05 |
| Steareth-2 | 2,00 |
| Steareth-21 | 2,50 |
| Alcool cétéaryl | 1,00 |
| Caprylate de propylheptyl | 15,00 |
| Hydroxyde de sodium (30 % en solution) | 0,10 |
| Mélange de phénoxyéthanol, chlorphenesin, acide benzoïque, Butylène glycol, acide sorbique (Germazide ^{™} PBS) | 1,25 |
| Mélange de polyacrylate-X, d'isohexadecane et de polysorbate 60 (Sepigel^{™} SMS 60) | 4,00 |
| Eau | Qsp 100 |

### 5b) Composition cosmétique contenant un extrait de Lythrum salicaria selon l'invention

| | |
|---|---|
| Ingrédient cosmétique* | 3,00 |
| EDTA | 0,05 |
| Steareth-2 | 2,00 |
| Steareth-21 | 2,50 |
| Alcool cétéaryl | 1,00 |
| Caprylate de propylheptyl | 15,00 |
| Hydroxyde de sodium (30 % en solution) | 0,10 |
| Mélange de phénoxyéthanol, chlorphenesin, acide benzoïque, butylène glycol, acide sorbique (Germazide ^{™} PBS) | 1,25 |
| Mélange de polyacrylate-X, d'isohexadecane et de polysorbate 60 (Sepigel^{™} SMS 60) | 4,00 |
| Eau | Qsp 100 |

| | |
|---|---|
| *L'ingrédient cosmétique est ici préparé selon l'exemple 4b ou 4c précédent. Les quantités indiquées sont en pourcentage en poids par rapport au poids total de la composition. | |

### Exemple 6 : Exemple d'une composition dermatologique sous forme de pommade contenant l'extrait selon l'invention.

La composition ci-après est préparée selon des méthodes connues de l'homme du métier, en particulier s'agissant des différentes phases à mélanger entre elles.

Les quantités indiquées sont en pourcentage en poids par rapport au poids total de la composition.

| | |
|---|---|
| Ingrédient * | 3,00 |
| Excipient : | |
| Polyéthylène basse densité | 5,50 |
| Paraffine liquide | Qsp 100 |

| | |
|---|---|
| * L'ingrédient est préparé selon l'exemple 4b ou 4c précédent. L'ingrédient selon l'invention est ici stérilisé puis séché avant d'être incorporé dans la composition sous forme de pommade. | |

## Revendications

1. Utilisation non thérapeutique d'un extrait de *Lythrum salicaria* pour maintenir et/ou augmenter la fermeté de la peau et/ou des muqueuses, l'extrait de *Lythrum salicaria* étant appliqué par voie topique sur une zone de peau et/ou de muqueuse saine.

2. Utilisation non thérapeutique selon la revendication 1, **caractérisée en ce que** l'extrait de *Lythrum salicaria* maintient et/ou augmente l'expression de collagène dans la peau et/ou les muqueuses, en particulier le collagène de type I.

3. Utilisation non thérapeutique selon l'une des revendications précédentes pour induire un effet amincissant au niveau de la peau et/ou diminuer, prévenir et /ou traiter les manifestations inesthétiques de la cellulite comme par exemple l'aspect peau d'orange de la peau ou la culotte de cheval et/ou pour la prévention et/ou le traitement de la cellulite.

4. Utilisation non thérapeutique d'un des dérivés de l'acide gallique choisi dans le groupe constitué par la vescalagine, la castalagine et leur mélange pour induire un effet amincissant au niveau de la peau et/ou diminuer, prévenir et/ou traiter les manifestations inesthétiques de la cellulite comme par exemple l'aspect peau d'orange de la peau ou la culotte de cheval et/ou pour la prévention et/ou le traitement de la cellulite.

5. Utilisation non thérapeutique selon l'une des revendications précédentes, **caractérisée en ce que** l'extrait de *Lythrum salicaria* et/ou un des dérivés de l'acide gallique choisi dans le groupe constitué par la vescalagine, la castalagine et leur mélange maintient et/ou augmente la lipolyse dans les adipocytes de la peau.

6. Utilisation selon l'une des revendications 1 à 3 ou 5, **caractérisée en ce que** l'extrait de *Lythrum salicaria* est un extrait obtenu par extraction aqueuse, préférentiellement par extraction avec une solution aqueuse contenant de l'éthanol, préférentiellement de 15 % à 40 % en poids par rapport au poids total de la solution aqueuse.

7. Utilisation selon l'une quelconque des revendications 1 à 3 ou 5 à 6, **caractérisée en ce que** l'extrait de *Lythrum salicaria* est un extrait des parties aériennes de la plante.

8. Utilisation selon l'une quelconque des revendications 1 à 3 ou 5 à 7, **caractérisée en ce que** l'extrait de *Lythrum salicaria* est obtenu par extraction d'une quantité de 0,1% à 10% en poids de matière sèche d'au moins une partie de la plante de *Lythrum salicaria* par rapport au poids total du mélange solvant/plante (p/p), préférentiellement d'une quantité de 1% à 10% en poids, encore préférentiellement d'une quantité de 5% ou 10% en poids.

9. Utilisation selon l'une quelconque des revendications 1 à 8 **caractérisée en ce que** l'extrait de *Lythrum salicaria* et/ou un des dérivés de l'acide gallique choisi dans le groupe constitué par la vescalagine, la castalagine et leur mélange est solubilisé dans une solution aqueuse comprenant de l'hexylène glycol et du caprylyl glycol ou leur mélange.

10. Utilisation selon l'une quelconque des revendications 1 à 3 ou 5 à 9, **caractérisée en ce que** l'extrait de Lythrum salicaria comprend au moins 0,25 %, préférentiellement au moins 0,5 %, encore préférentiellement au moins 1 % en poids de vescalagine et/ou de castalagine par rapport au poids total de l'extrait.

11. Utilisation selon l'une quelconque des revendications 1 à 3 ou 5 à 10 **caractérisée en ce que** l'extrait de Lythrum salicaria comprend au moins 20 % en poids de maltodextrine par rapport au poids total de l'extrait et de la maltodextrine, préférentiellement au moins 50 %, encore préférentiellement au moins 70 % en poids.

12. Utilisation selon l'une quelconque des revendications 1 à 11, **caractérisée en ce que** l'extrait de *Lythrum salicaria* et/ou un des dérivés de l'acide gallique, choisi dans le groupe constitué par la vescalagine, la castalagine et leur mélange est présent dans une composition cosmétique comprenant au moins un excipient cosmétiquement acceptable.

13. Utilisation selon la revendication 12, **caractérisée en ce que**, l'extrait de *Lythrum salicaria* est présent dans la composition à une concentration de 1×10⁻⁴ % à 10%, préférentiellement de 1×10⁻⁴ % à 5 %, et encore préférentiellement de 1×10⁻³ % à 3% en poids, par rapport au poids total de la composition.

14. Utilisation selon la revendication 12, **caractérisée en ce qu'**un des dérivés de l'acide gallique choisi dans le groupe constitué par la vescalagine, la castalagine et leur mélange est présent dans la composition à une concentration finale totale en molécules de 2,5×10⁻⁷ à 0,1 %, préférentiellement de 5×10⁻⁷ à 0,05 %, encore préférentiellement de 1×10⁻⁵ à 0,03 % en poids, par rapport au poids total de la composition.

15. Utilisation selon l'une quelconque des revendications 12 à 14, **caractérisée en ce que** la composition cosmétique est choisie parmi une solution, aqueuse ou huileuse, une crème ou un gel aqueux ou un gel huileux, notamment un gel douche, un shampoing, un lait, une émulsion, une microémulsion ou une nanoémulsion, notamment huile-dans-eau ou eau-dans-huile ou multiple ou siliconée, un masque, un sérum, une lotion, un savon liquide, un pain non dermatologique, une pommade, une mousse, un patch, préférentiellement une crème ou un sérum.

16. Procédé de soin non thérapeutique **caractérisé en ce qu'**il comprend l'application par voie topique sur au moins une zone de peau et/ou de muqueuse saine, d'un extrait de *Lythrum salicaria* ou d'une composition cosmétique comprenant un tel extrait pour maintenir et/ou augmenter la fermeté de la peau et/ou des muqueuses.

17. Procédé de soin non thérapeutique selon la revendication 16, **caractérisé en ce que** l'extrait de *Lythrum salicaria* maintient et/ou augmente l'expression de collagène, en particulier du collagène de type I.

18. Procédé de soin non thérapeutique selon l'une des revendications 16 ou 17 pour maintenir et/ou augmenter la lipolyse dans les adipocytes de la peau, pour induire un effet amincissant au niveau de la peau et/ou diminuer, prévenir et/ou traiter les manifestations inesthétiques de la cellulite comme l'aspect peau d'orange de la peau et/ou la culotte de cheval et/ou pour la prévention et/ou le traitement de la cellulite.

19. Procédé de soin non thérapeutique selon l'une quelconque des revendications 16 à 18, **caractérisé en ce que** l'extrait de *Lythrum salicaria* est tel que défini dans les revendications 6 à 13.

20. Procédé de soin non thérapeutique **caractérisé en ce qu'**il comprend l'application par voie topique sur au moins une zone de peau et/ou de muqueuse saine, d'un des dérivés de l'acide gallique choisi dans le groupe constitué par la vescalagine, la castalagine et leur mélange ou d'une composition cosmétique comprenant un des dérivés de l'acide gallique choisi dans le groupe constitué par la vescalagine, la castalagine et leur mélange, pour maintenir et/ou augmenter la lipolyse dans les adipocytes de la peau, pour induire un effet amincissant au niveau de la peau et/ou diminuer, prévenir et/ou traiter les manifestations inesthétiques de la cellulite comme l'aspect peau d'orange de la peau et/ou la culotte de cheval.

21. Procédé de soin non thérapeutique selon l'une quelconque des revendications 16 à 20, **caractérisé en ce que** la zone de peau et/ou de muqueuse saine est choisie parmi le ventre, les bras, les avant-bras, les genoux, le buste, les cuisses, les hanches, les fesses, le cou, la taille et/ou le visage, et préférentiellement le corps, en particulier, sur une zone de peau comprenant de la cellulite et/ou ses manifestations inesthétiques telles que la peau d'orange ou la culotte de cheval et/ou sur une zone de peau manquant de fermeté en raison de variations physiologiques non pathologiques telles qu'un changement de régime alimentaire, les variations hormonales, en particulier durant la puberté, la grossesse, la ménopause mais aussi une mauvaise alimentation, la sédentarité et plus particulièrement chez la femme.

22. Extrait de *Lythrum salicaria* et/ou dérivé de l'acide gallique choisi dans le groupe constitué par la vescalagine, la castalagine et leur mélange ou composition dermatologique le comprenant avec un excipient dermatologiquement acceptable pour son utilisation, avantageusement par voie topique, pour le traitement et/ou la prévention des télangiectasies.

23. Extrait ou composition pour son utilisation selon la revendication 22 **caractérisé en ce que** l'extrait est tel que défini dans les revendications 6 à 11.

## Patentansprüche

1. Nicht-therapeutische Verwendung eines *Lythrum salicaria*-Extrakts zur Aufrechterhaltung und/oder Steigerung der Straffheit der Haut und/oder der Schleimhäute, wobei der *Lythrum salicaria-Extrakt* topisch auf einen gesunden Haut- und/oder Schleimhautbereich aufgebracht wird.

2. Nicht-therapeutische Verwendung der Anspruch 1, **dadurch gekennzeichnet, dass** der *Lythrum salicaria-*Extrakt die Expression von Kollagen in der Haut und/oder den Schleimhäuten, insbesondere von Kollagen Typ I, aufrechterhält und/oder steigert.

3. Nicht-therapeutische Verwendung nach einem der vorhergehenden Ansprüche zum Herbeiführen einer Schlankheitswirkung an der Haut und/oder zur Verringerung, Vorbeugung und/oder Behandlung der unästhetischen Erscheinungsformen von Cellulitis, wie zum Beispiel das Orangenhaut-Erscheinungsbild der Haut oder Reiterhosen, und/oder zur Vorbeugung und/oder Behandlung von Cellulitis.

4. Nicht-therapeutische Verwendung eines der Derivate der Gallussäure, ausgewählt aus der aus Vescalagin, Castalagin und deren Gemisch bestehenden Gruppe, zum Herbeiführen einer Schlankheitswirkung an der Haut und/oder zur Verringerung, Vorbeugung und/oder Behandlung der unästhetischen Erscheinungsformen von Cellulitis, wie zum Beispiel das Orangenhaut-Erscheinungsbild der Haut oder Reiterhosen, und/oder zur Vorbeugung und/oder Behandlung von Cellulitis.

5. Nicht-therapeutische Verwendung nach einem der vorhergehenden Ansprüche, **dadurch gekennzeichnet, dass** der *Lythrum salicaria-Extrakt* und/oder eines der Derivate der Gallussäure, ausgewählt aus der aus Vescalagin, Castalagin und deren Gemisch bestehenden Gruppe, die Lipolyse in den Adipozyten der Haut aufrechterhält und/oder steigert.

6. Verwendung nach einem der Ansprüche 1 bis 3 oder 5, **dadurch gekennzeichnet, dass** der *Lythrum salicaria-*Extrakt ein durch wässrige Extraktion gewonnener Extrakt ist, vorzugsweise durch Extraktion mit einer wässrigen Lösung, die Ethanol, vorzugsweise zu 15 Gew.-% bis 40 Gew.-%, bezogen auf das Gesamtgewicht der wässrigen Lösung, enthält.

7. Verwendung nach einem der Ansprüche 1 bis 3 oder 5 bis 6, **dadurch gekennzeichnet, dass** der *Lythrum salicaria-Extrakt* ein Extrakt aus den oberirdischen Teilen der Pflanze ist.

8. Verwendung nach einem der Ansprüche 1 bis 3 oder 5 bis 7, **dadurch gekennzeichnet, dass** der *Lythrum salicaria-Extrakt* durch Extraktion einer Menge von 0,1 Gew.-% bis 10 Gew.-% Trockenmasse mindestens eines Teils der *Lythrum salicaria*-Pflanze, bezogen auf das Gesamtgewicht des Gemischs von Lösungsmittel/Pflanze (Gew./Gew.), vorzugsweise einer Menge von 1 Gew.-% bis 10 Gew.-%, ebenfalls bevorzugt einer Menge von 5 Gew.-% oder 10 Gew.-%, gewonnen wird.

9. Verwendung nach einem der Ansprüche 1 bis 8, **dadurch gekennzeichnet, dass** der *Lythrum salicaria-*Extrakt und/oder eines der Derivate der Gallussäure, ausgewählt aus der aus Vescalagin, Castalagin und deren Gemisch bestehenden Gruppe, in einer wässrigen Lösung solubilisiert wird, die Hexylenglykol und Caprylylglykol oder deren Gemisch umfasst.

10. Verwendung nach einem der Ansprüche 1 bis 3 oder 5 bis 9, **dadurch gekennzeichnet, dass** der *Lythrum salicaria-Extrakt* mindestens 0,25 Gew.-%, vorzugsweise mindestens 0,5 Gew.-%, ebenfalls bevorzugt mindestens 1 Gew.-% Vescalagin und/oder Castalagin, bezogen auf das Gesamtgewicht des Extrakts, umfasst.

11. Verwendung nach einem der Ansprüche 1 bis 3 oder 5 bis 10, **dadurch gekennzeichnet, dass** der *Lythrum salicaria-Extrakt* mindestens 20 Gew.-% Maltodextrin, bezogen auf das Gesamtgewicht von Extrakt und Maltodextrin, vorzugsweise mindestens 50 Gew.-%, ebenfalls bevorzugt mindestens 70 Gew.-%, umfasst.

12. Verwendung nach einem der Ansprüche 1 bis 11, **dadurch gekennzeichnet, dass** der *Lythrum salicaria-*Extrakt und/oder eines der Derivate der Gallussäure, ausgewählt aus der aus Vescalagin, Castalagin und deren Gemisch bestehenden Gruppe, in einer Kosmetikzusammensetzung vorliegt, die mindestens einen kosmetisch akzeptablen Exzipienten umfasst.

13. Verwendung nach Anspruch 12, **dadurch gekennzeichnet, dass** der *Lythrum salicaria-Extrakt* in der Zusammensetzung in einer Konzentration von 1×10⁻⁴ Gew.-% bis 10 Gew.-%, vorzugsweise von 1×10⁻⁴ Gew.-% bis 5 Gew.-% und ebenfalls bevorzugt von 1×10⁻³ Gew.-% bis 3 Gew.-%, bezogen auf das Gesamtgewicht der Zusammensetzung, vorliegt.

14. Verwendung nach Anspruch 12, **dadurch gekennzeichnet, dass** eines der Derivate der Gallussäure, ausgewählt aus der aus Vescalagin, Castalagin und deren Gemisch bestehenden Gruppe, in der Zusammensetzung in einer Gesamt-Endkonzentration an Molekülen von 2,5×10⁻⁷ bis 0,1 Gew.-%, vorzugsweise von 5×10⁻⁷ bis 0,05 Gew.-%, ebenfalls bevorzugt von 1×10⁻⁵ bis 0,03 Gew.-%, bezogen auf das Gesamtgewicht der Zusammensetzung, vorliegt.

15. Verwendung nach einem der Ansprüche 12 bis 14, **dadurch gekennzeichnet, dass** die Kosmetikzusammensetzung aus einer wässrigen oder öligen Lösung, einer Creme oder einem wässrigen Gel oder einem öligen Gel, insbesondere einem Duschgel, einem Shampoo, einer Milch, einer Emulsion, einer, insbesondere Öl-in-Wasser- oder Wasser-in-Öl- oder Mehrfach- oder Silikon-, Mikroemulsion oder Nanoemulsion, einer Maske, einem Serum, einer Lotion, einer Flüssigseife, einem nichtdermatologischen Waschstück, einer Salbe, einem Schaum, einem Pflaster, vorzugsweise einer Creme oder einem Serum ausgewählt ist.

16. Nicht-therapeutisches Pflegeverfahren, **dadurch gekennzeichnet, dass** es das topische Aufbringen auf mindestens einen gesunden Haut- und/oder Schleimhautbereich eines *Lythrum* salicaria-Extrakts oder einer einen solchen Extrakt umfassenden Kosmetikzusammensetzung zur Aufrechterhaltung und/oder Steigerung der Straffheit der Haut und/oder der Schleimhäute umfasst.

17. Nicht-therapeutisches Pflegeverfahren nach Anspruch 16, **dadurch gekennzeichnet, dass** der *Lythrum salicaria*-Extrakt die Expression von Kollagen, insbesondere von Kollagen Typ I, aufrechterhält und/oder steigert.

18. Nicht-therapeutisches Pflegeverfahren nach einem der Ansprüche 16 oder 17 zum Aufrechterhalten und/oder Steigern der Lipolyse in den Adipozyten der Haut, zum Herbeiführen einer Schlankheitswirkung an der Haut und/oder zur Verringerung, Vorbeugung und/oder Behandlung der unästhetischen Erscheinungsformen von Cellulitis, wie das Orangenhaut-Erscheinungsbild der Haut und/oder Reiterhosen, und/oder zur Vorbeugung und/oder Behandlung von Cellulitis.

19. Nicht-therapeutisches Pflegeverfahren nach einem der Ansprüche 16 bis 18, **dadurch gekennzeichnet, dass** der *Lythrum salicaria*-Extrakt wie in den Ansprüchen 6 bis 13 definiert ist.

20. Nicht-therapeutisches Pflegeverfahren, **dadurch gekennzeichnet, dass** es das topische Aufbringen auf mindestens einen gesunden Haut- und/oder Schleimhautbereich eines der Derivate der Gallussäure, ausgewählt aus der aus Vescalagin, Castalagin und deren Gemisch bestehenden Gruppe, oder einer Kosmetikzusammensetzung, die eines der Derivate der Gallussäure, ausgewählt aus der aus Vescalagin, Castalagin und deren Gemisch bestehenden Gruppe, umfasst, zum Aufrechterhalten und/oder Steigern der Lipolyse in den Adipozyten der Haut, zum Herbeiführen einer Schlankheitswirkung an der Haut und/oder zur Verringerung, Vorbeugung und/oder Behandlung der unästhetischen Erscheinungsformen von Cellulitis, wie das Orangenhaut-Erscheinungsbild der Haut und/oder Reiterhosen, umfasst.

21. Nicht-therapeutisches Pflegeverfahren nach einem der Ansprüche 16 bis 20, **dadurch gekennzeichnet, dass** der gesunde Haut- und/oder Schleimhautbereich aus dem Bauch, den Armen, den Unterarmen, den Knien, der Brust, den Oberschenkeln, den Hüften, dem Gesäß, dem Hals, der Taille und/oder dem Gesicht und vorzugsweise dem Körper ausgewählt ist, insbesondere auf einen Hautbereich, der Cellulitis und/oder deren unästhetische Erscheinungsformen, wie Orangenhaut oder Reiterhosen, aufweist, und/oder auf einen Hautbereich, dem es aufgrund nicht-pathologischer physiologischer Veränderungen, wie einer Änderung der Ernährungsweise, Hormonschwankungen, insbesondere während Pubertät, Schwangerschaft, Menopause, aber auch einer schlechten Ernährung, sitzender Tätigkeit, und ganz besonders bei Frauen, an Straffheit fehlt.

22. *Lythrum salicaria-Extrakt* und/oder Derivat der Gallussäure, ausgewählt aus der aus Vescalagin, Castalagin und deren Gemisch bestehenden Gruppe, oder dermatologische Zusammensetzung, die dies mit einem dermatologisch akzeptablen Exzipienten enthält, für die, vorzugsweise topische, Verwendung zur Behandlung und/oder Vorbeugung von Teleangiektasien.

23. Extrakt oder Zusammensetzung zur Verwendung nach Anspruch 22, **dadurch gekennzeichnet, dass** der Extrakt wie in den Ansprüchen 6 bis 11 definiert ist.

## Claims

1. Non-therapeutic use of a *Lythrum salicaria* extract for maintaining and/or increasing the firmness of the skin and/or of the mucous membranes, the *Lythrum salicaria* extract being applied topically to a healthy area of skin and/or of mucous membrane.

2. Non-therapeutic use according to Claim 1, **characterized in that** the *Lythrum salicaria* extract maintains and/or increases collagen expression in the skin and/or the mucous membranes, more particularly type I collagen.

3. Non-therapeutic use according to either of the preceding claims for inducing a slimming effect at the level of the skin and/or decreasing, preventing and/or treating the unaesthetic manifestations of cellulite, for instance the orange peel appearance of the skin or the jodhpur thigh appearance, and/or for preventing and/or treating cellulite.

4. Non-therapeutic use of one of the gallic acid derivatives selected from the group consisting of vescalagin, castalagin and a mixture thereof for inducing a slimming effect at the level of the skin and/or decreasing, preventing and/or treating the unaesthetic manifestations of cellulite, for instance the orange peel appearance of the skin or the jodhpur thigh appearance, and/or for preventing and/or treating cellulite.

5. Non-therapeutic use according to one of the preceding claims, **characterized in that** the *Lythrum salicaria* extract and/or one of the gallic acid derivatives selected from the group consisting of vescalagin, castalagin and a mixture thereof maintains and/or increases lipolysis in skin adipocytes.

6. Use according to one of Claims 1 to 3 or 5, **characterized in that** the *Lythrum salicaria* extract is an extract obtained by aqueous extraction, preferentially by extraction with an aqueous solution containing ethanol, preferentially from 15% to 40% by weight relative to the total weight of the aqueous solution.

7. Use according to any one of Claims 1 to 3 or 5 to 6, **characterized in that** the *Lythrum salicaria* extract is an extract of the aerial parts of the plant.

8. Use according to any one of Claims 1 to 3 or 5 to 7, **characterized in that** the *Lythrum salicaria* extract is obtained by extraction of an amount of from 0.1% to 10% by weight of dry matter of at least one part of the *Lythrum salicaria* plant relative to the total weight of the solvent/plant mixture (w/w), preferentially of an amount of from 1% to 10% by weight, more preferentially of an amount of 5% or 10% by weight.

9. Use according to any one of Claims 1 to 8, **characterized in that** the *Lythrum salicaria* extract and/or one of the gallic acid derivatives selected from the group consisting of vescalagin, castalagin and a mixture thereof is solubilized in an aqueous solution comprising hexylene glycol and caprylyl glycol or a mixture thereof.

10. Use according to any one of Claims 1 to 3 or 5 to 9, **characterized in that** the *Lythrum salicaria* extract comprises at least 0.25%, preferentially at least 0.5%, more preferentially at least 1% by weight of vescalagin and/or of castalagin relative to the total weight of the extract.

11. Use according to any one of Claims 1 to 3 or 5 to 10, **characterized in that** the *Lythrum salicaria* extract comprises at least 20% by weight of maltodextrin relative to the total weight of the extract and of the maltodextrin, preferentially at least 50%, more preferentially at least 70% by weight.

12. Use according to any one of Claims 1 to 11, **characterized in that** the *Lythrum salicaria* extract and/or one of the gallic acid derivatives selected from the group consisting of vescalagin, castalagin and a mixture thereof is present in a cosmetic composition comprising at least one cosmetically acceptable excipient.

13. Use according to Claim 12, **characterized in that** the *Lythrum salicaria* extract is present in the composition at a concentration of from 1×10⁻⁴% to 10%, preferentially from 1×10⁻⁴% to 5%, and more preferentially from 1×10⁻³% to 3% by weight, relative to the total weight of the composition.

14. Use according to Claim 12, **characterized in that** one of the gallic acid derivatives selected from the group consisting of vescalagin, castalagin and a mixture thereof is present in the composition at a total final concentration of molecules of from 2.5×10⁻⁷ to 0.1%, preferentially from 5×10⁻⁷ to 0.05%, more preferentially from 1×10⁻⁵ to 0.03% by weight, relative to the total weight of the composition.

15. Use according to any one of Claims 12 to 14, **characterized in that** the cosmetic composition is chosen from an aqueous or oily solution, a cream or an aqueous gel or an oily gel, in particular a shower gel, a shampoo, a milk, an emulsion, a microemulsion or a nanoemulsion, which is in particular oil-in-water or water-in-oil or multiple or silicone-based, a mask, a serum, a lotion, a liquid soap, a non-dermatological bar, an ointment, a foam and a patch, preferentially a cream or a serum.

16. Non-therapeutic care process, **characterized in that** it comprises the application topically, to at least one area of skin and/or of mucous membrane, of a *Lythrum salicaria* extract or of a cosmetic composition comprising such an extract for maintaining and/or increasing the firmness of the skin and/or of the mucous membranes.

17. Non-therapeutic care process according to Claim 16, **characterized in that** the *Lythrum salicaria* extract maintains and/or increases collagen expression, more particularly of type I collagen.

18. Non-therapeutic care process according to one of Claims 16 and 17 for maintaining and/or increasing lipolysis in skin adipocytes, for inducing a slimming effect at the level of the skin and/or decreasing, preventing and/or treating the unaesthetic manifestations of cellulite, for instance the orange peel appearance of the skin and/or the jodhpur thigh appearance, and/or for preventing and/or treating cellulite.

19. Non-therapeutic care process according to any one of Claims 16 to 18, **characterized in that** the *Lythrum salicaria* extract is as defined in Claims 6 to 13.

20. Non-therapeutic care process, **characterized in that** it comprises the application topically, to at least one area of skin and/or of mucous membrane, of one of the gallic acid derivatives selected from the group consisting of vescalagin, castalagin and a mixture thereof or of a cosmetic composition comprising one of the gallic acid derivatives selected from the group consisting of vescalagin, castalagin and a mixture thereof for maintaining and/or increasing lipolysis in skin adipocytes, for inducing a slimming effect at the level of the skin and/or decreasing, preventing and/or treating the unaesthetic manifestations of cellulite, for instance the orange peel appearance of the skin and/or the jodhpur thigh appearance.

21. Non-therapeutic care process according to any one of Claims 16 to 20, **characterized in that** the area of skin and/or of mucous membrane is chosen from the stomach, the arms, the forearms, the knees, the bust, the thighs, the hips, the buttocks, the neck, the waist and/or the face, and preferentially the body, in particular on an area of skin comprising cellulite and/or the unaesthetic manifestations thereof, such as the orange peel appearance or the jodhpur thigh appearance, and/or on an area of skin which lacks firmness owing to non-pathological physiological variations, such as a change in diet, hormonal variations, in particular during puberty, pregnancy or menopause, but also a poor diet, or a sedentary lifestyle, and more particularly in women.

22. *Lythrum salicaria* extract and/or gallic acid derivative selected from the group consisting of vescalagin, castalagin and a mixture thereof or dermatological composition comprising it with a dermatologically acceptable excipient, for use thereof, advantageously topically, for the treatment and/or prevention of telangiectasia.

23. Extract or composition for use thereof according to Claim 22, **characterized in that** the extract is as defined in Claims 6 to 11.
